# EUROPEAN PATENT APPLICATION

(11) **EP 4 549 464 A1**
(43) Date of publication of application: **07.05.2025**
(21) Application number: 23830024.8
(22) Date of filing: 19.06.2023
(51) Int. Cl.: C07K 16/28, C07K 19/00, A61K 39/395, A61P 35/00, A61K 47/68, C12N 15/13, C12N 5/10

(54) **METHOD FOR PREPARING HER2 NANOBODY AND CONJUGATE, AND USE THEREOF**

(30) Priority: 30.06.2022 CN 202210770514; 04.05.2023 WO PCT/CN2023/092107
(71) Applicant: Fudan University, Shanghai 200433 (CN)
(72) Inventor: YU, Ke, Shanghai 200433 (CN); WANG, Yue, Shanghai 200433 (CN); MENG, Tao, Shanghai 200433 (CN); LIU, Liang, Shanghai 200433 (CN)
(74) Representative: Mewburn Ellis LLP
(86) International application number: PCT/CN2023/101129
(87) International publication number: WO 2024/001844

(57) **Abstract**

Provided are a new nanobody targeting human HER2/ErbB2 (HER2-Nanobody, HER2-Nb), a method for preparing same, and use thereof. The monoclonal nanobody efficiently and specifically binds to a purified HER2 protein and HER2 on the surface of a tumor cell, blocks a signal pathway of tumor HER2, and can be used for preparing a therapeutic antibody, an antibody-fusion protein or an antibody-drug conjugate targeting HER2-positive tumors.

## Description

### TECHNICAL FIELD

The present invention relates to the field of medicine, and in particular to a method for preparing HER2 nanobody and conjugate, and use thereof.

### BACKGROUND

Human epidermal growth factor receptor-2 (Her2/ErbB2), also known as Her2/Neu, ErbB-2, or CD340, is a protein encoded by human Her2 gene. HER2 is a receptor tyrosine kinase (RTK) belonging to the epidermal growth factor receptor (EGFR/ErbB) family. It consists of 1255 amino acids, including four extracellular domains (I, II, III and IV) and one transmembrane domain. It also has a tyrosine kinase activity domain and a carboxyl terminal tail containing tyrosine residues with intracellular signal molecular anchor site. The molecular weight of HER2 is about 185kD.

HER2 is overexpressed in breast cancer, ovarian cancer, prostate cancer, gastric cancer, lung cancer and other epitherlial cell-derived malignant tumors while low-expressed or unexpressed in normal tissues. The amplification or overexpression of HER2 plays an important role in the pathogenesis and development of some invasive breast cancer, and it has become an important marker and therapeutic target of breast cancer. HER2 targeted therapy significantly improved the prognosis of HER2 positive breast cancer patients and gastric cancer patients. At present, several anti HER2 targeted drugs have been approved for the treatment of breast cancer: humanized recombinant monoclonal antibodies, Trastuzumab and Pertuzumab, both of which are human monoclonal antibodies against HER2, and their combination with chemotherapy has been approved as the first-line treatment of metastatic breast cancer. Antibody conjugated drugs (ADCs) combine the strong cytotoxicity of small molecule drugs with the precision of monoclonal antibodies and good pharmacokinetic characteristics, making HER2 targeting ADCs become a very promising direction for the treatment of HER2 positive cancer. However, breast cancer is prone to brain metastasis, second only to lung cancer, accounting for 15% -20% of all brain metastases. In recent years, the incidence of brain metastasis of breast cancer is increasing. On the one hand, with the improvement of comprehensive treatment strategy for breast cancer, the survival period of patients has been prolonged, on the other hand, the development of magnetic resonance imaging and other imaging technologies has increased the detection rate of brain metastases. Breast cancer with brain metastasis is mainly triple negative breast cancer (TNBC) and HER2 positive breast cancer. About 25% -50% of HER2 positive advanced breast cancer will be accompanied by brain metastasis. Once patients with brain metastasis occur, their life expectancy will be shortened. Retrospective studies show that the median overall survival (OS) of breast cancer patients with brain metastases is only 6.9 months, and the 1-year OS rate is only 35%, and the 2-year OS rate is only 17%, and the quality of life of patients with brain metastasis will be reduced. Previous studies have shown that patients with brain metastases have a poor response to medical therapy, one of the reasons is that many macromolecular drugs can not penetrate the blood brain barrier (BBB), and conventional macromolecular drugs can not reach the effective therapeutic concentration, which has brought challenges to the treatment of brain metastasis of breast cancer.

Conventional antibodies have played a huge role in the field of cancer, especially ushering in the era of targeted therapy. Conventional antibodies contain Fc domain and can kill cancer cells through ADCC, CDC activity, etc. Conventional monoclonal antibodies have a molecular weight of up to 150 kd, complex production processes and high production cost. The size of conventional monoclonal antibodies makes it difficult to penetrate tissue, resulting in low effective concentration in the tumor area and insufficient therapeutic effect. Moreover, conventional monoclonal antibodies have high immunogenicity and are always difficult to achieve the original affinity after modification. Moreover, the long-term development of fully humanized conventional antibodies, high production costs, insufficient stability and many other factors limit its application and popularity in the clinic. Nanobodies are currently the smallest antibody binding domain with molecular weight of 1/10 of that of a conventional antibody. In addition to the high binding affinity of conventional monoclonal antibodies, nanobodies also have some unique functional characteristics, such as low molecular weight, strong stability, good solubility, easy expression, weak immunogenicity, strong penetration, simple humanization, low production cost etc. Nanobodies overcome shortcomings of conventional antibodies, such as long development cycle, low stability and harsh preservation conditions. Due to their unique structure, as a targeted carrier, nanobodies can penetrate the tumor tissue binding target quickly and specifically, while the non-binding nanobodies can be quickly removed from the blood, thereby improving the safety window of drug conjugates.

The ADC drug Enhertu (DS8201a) targeting HER2 shows superior therapeutic effects in breast cancer, gastric cancer, lung cancer and colorectal cancer, but the side effects and drug resistance caused by the drug, such as interstitial pneumonia (ILD), are also unmet clinical needs that need to be addressed at present. In addition, HER2 abnormalities are present in many tumors, including HER2 gene mutations, deletions, amplifications, and HER2 protein overexpression. In addition to breast cancer, HER2 overexpression also exists in other solid tumors, such as adenocarcinoma at the junction of stomach and gastroesophagus, cholangiocarcinoma, colorectal cancer, non-small cell lung cancer, and bladder cancer. Chemotherapy drugs coupled with nanobodies targeting HER2 are expected to further improve the clinical response of HER2 positive but low to moderate expression populations, as well as the clinical application of specific indications such as HER2 positive breast cancer, lung cancer patients with brain metastases, etc.

To sum up, the development of nanobodies and their drug conjugate targeting HER2 can provide new therapeutic strategies for HER2 positive tumor patients.

### SUMMARY OF THE INVENTION

The object of the present invention is to provide a method for preparing HER2 nanobody and nanobody-drug conjugate, and use thereof.

The present invention provides a HER2 nanobody (HER2-Nb), and the HER2-Nb of the present invention can bind purified recombinant HER2 protein and HER2 receptor on the surface of tumor cells high efficiently and specifically, and block tumor HER2 signal. The nanobody of the present invention can be used to prepare nanobody drugs, antibody fusion proteins, and antibody-drug conjugates for targeting HER2-positive tumors.

In the first aspect of the present invention, it provides a nanobody targeting HER2, and the complementary determining region CDR of the VHH chain in the nanobody is selected from one or more of the following groups:
(1) CDR1 as shown in SEQ ID NO. 1,
   CDR2 as shown in SEQ ID NO. 2, and
   CDR3 as shown in SEQ ID NO. 3;
      or,
(2) CDR1 as shown in SEQ ID NO. 5,
   CDR2 as shown in SEQ ID NO. 6, and
   CDR3 as shown in SEQ ID NO. 7;
      or,
(3) CDR1 as shown in SEQ ID NO. 9,
   CDR2 as shown in SEQ ID NO. 10, and
   CDR3 as shown in SEQ ID NO. 11;
      or,
(4) CDR1 as shown in SEQ ID NO. 13,
   CDR2 as shown in SEQ ID NO. 14, and
   CDR3 as shown in SEQ ID NO. 15;
      or,
(5) CDR1 as shown in SEQ ID NO. 17,
   CDR2 as shown in SEQ ID NO. 18, and
   CDR3 as shown in SEQ ID NO. 19;
      or,
(6) CDR1 as shown in SEQ ID NO. 21,
   CDR2 as shown in SEQ ID NO. 22, and
   CDR3 as shown in SEQ ID NO. 23.

In another preferred embodiment, any one of the above amino acid sequences further includes a derivative sequence that is optionally added, deleted, modified and/or substituted with at least one amino acid and can retain the ability to bind to HER2.

In another preferred embodiment, the region CDR of the VHH chain in the nanobody comprises an amino acid sequence having a sequence similarity of at least 80%, preferably at least 90%, more preferably at least 95%, even more preferably at least 99%, to any one of SEQ ID NOs. 1-36.

In another preferred embodiment, the amino acid sequence of the region CDR of the VHH chain in the nanobody comprises one or more amino acid substitutions, preferably conservative amino acid substitutions, compared to any one of SEQ ID NOs. 1-36.

In another preferred embodiment, the VHH chain comprises CDR1, CDR2, and CDR3 selected from the following group:
(1) the complementary determining regions CDR1, CDR2, and CDR3 as shown in SEQ ID NO.1, SEQ ID NO.2, and SEQ ID NO.3 (corresponding to the CDRs of nanobody 1-C10); or
(2) the complementary determining regions CDR1, CDR2, and CDR3 as shown in SEQ ID NO.5, SEQ ID NO.6, and SEQ ID NO.7 (corresponding to the CDRs of nanobody 2-C07); or
(3) the complementary determining regions CDR1, CDR2, and CDR3 as shown in SEQ ID NO.9, SEQ ID NO.10, and SEQ ID NO.11 (corresponding to the CDRs of nanobody 1-B05); or
(4) the complementary determining regions CDR1, CDR2, and CDR3 as shown in SEQ ID NO.13, SEQ ID NO.14, and SEQ ID NO.15 (corresponding to the CDRs of nanobody 1-C09); or
(5) the complementary determining regions CDR1, CDR2, and CDR3 as shown in SEQ ID NO.17, SEQ ID NO.18, and SEQ ID NO.19 (corresponding to the CDRs of nanobody 2-F03); or
(6) the complementary determining regions CDR1, CDR2, and CDR3 as shown in SEQ ID NO.20, SEQ ID NO.21, and SEQ ID NO.22 (corresponding to the CDRs of nanobody 1-G07).

In another preferred embodiment, the CDR1, CDR2, and CDR3 are separated by the framework regions FR1, FR2, FR3, and FR4 of the VHH chain.

In another preferred embodiment, the VHH chain of the nanobody further comprises a framework region (FR).

In another preferred embodiment, the framework region FR is derived from human, mouse, rabbit, or camel.

In another preferred embodiment, the framework region FR comprises FR regions is derived from human, mouse, or camel.

In another preferred embodiment, the VHH chain in the nanobody targeting HER2 has an amino acid sequence as shown in SEQ ID NO.4, SEQ ID NO.8, SEQ ID NO.12, SEQ ID NO.16, SEQ ID NO.20, or SEQ ID NO.24.

In the second aspect of the present invention, it provides an antibody targeting HER2, the antibody comprises one or more VHH chains in the nanobody targeting HER2 of the first aspect of the present invention.

In another preferred embodiment, the VHH chain in the nanobody targeting HER2 has an amino acid sequence as shown in SEQ ID NO.4, SEQ ID NO.8, SEQ ID NO.12, SEQ ID NO.16, SEQ ID NO.20, or SEQ ID NO.24.

In another preferred embodiment, the antibody is a monomer, bivalent antibody, tetravalent antibody, and/or multivalent antibody.

In another preferred embodiment, the antibody includes an animal antibody, a humanized antibody, a chimeric antibody or chimeric antigen receptor antibody (CAR).

In another preferred embodiment, the CDR region of the humanized antibody comprises 1, 2, or 3 amino acid changes.

In another preferred embodiment, the animal is a non-human mammal, preferably a mouse, sheep, or rabbit.

In another preferred embodiment, the antibody is a double chain antibody or a single chain antibody.

In another preferred embodiment, the antibody is a monoclonal antibody.

In another preferred embodiment, the antibody is a partially or fully humanized monoclonal antibody.

In another preferred embodiment, the number of added, deleted, modified and/or substituted amino acids does not exceed 40%, preferably 20%, more preferably 10% of the total number of amino acids in the initial amino acid sequence.

In another preferred embodiment, the number of added, deleted, modified and/or substituted amino acids is 1-7, preferably 1-3, and more preferably one.

In another preferred embodiment, the sequence obtained through addition, deletion, modification and/or substitution of at least one amino acid is an amino acid sequence with at least 80% homology.

In another preferred embodiment, the derivative sequence obtained through addition, deletion, modification and/or substitution of at least one amino acid has the function of inhibiting cell surface HER2 or recombinant HER2 protease.

In another preferred embodiment, the antibody is in the form of a drug conjugate.

In another preferred embodiment, the binding affinity EC₅₀ of the nanobody to the extracellular domain of human HER2 protein (HER2-ECD) by ELISA is 0.004-0.017 µg/mL (0.05-0.22 nM), or the KD values of affinity determined by ForteBio surface plasmon resonance (SPR) ranged from 4.61 nM to 23.24nM.

In another preferred embodiment, the antibody has one or more properties selected from the following group:
(a) specifically binding to the extracellular domain of HER2 receptor protein and regulating receptor activity;
(b) specifically binding to tumor cells, and/or HER2 of the immune/stromal cells in the tumor microenvironment;
(c) regulating the interaction of tumor cells with the stroma/immune microenvironment to promote the effect of tumor immunity;
(d) inhibiting tumor cell migration or metastasis;
(e) inhibiting tumor growth and improving the anti-tumor efficacy of combination drug therapy.

In the third aspect of the present invention, it provides a multispecific antibody, wherein the multispecific antibody comprises: the nanobody targeting HER2 of the first aspect of the present invention, or the antibody targeting HER2 of the second aspect of the present invention.

In another preferred embodiment, the multispecific antibody comprises a second antigen binding region targeting a target selected from the following group: EGFR, TGFβ, BCMA, B7H6, GUCY2C, DLL3, CD38, CD123, CD19, CD20, CD22, B7-H3, GPC3, CD73, PMSA, CD28, 4-1BB, OX40, CD40, CD27, CD3, CTLA4, PD1, PDL1, BCMA, GLP-1, Trop2, TIGIT, LAG-3, FGL1, TLR7, or a combination thereof.

In another preferred embodiment, the second antigen-binding region is a nanobody.

In another preferred embodiment, the multispecific antibody comprises one or more second antigen-binding regions.

In another preferred embodiment, the multispecific antibody further comprises an Fc segment of the antibody.

In another preferred embodiment, the antigen-binding region is an antibody or an antibody fragment, and the antibody fragment includes: (i) Fab fragment; (ii) F(ab')₂ fragment; (iii) Fd fragment; (iv) Fv fragment; (v) single-stranded Fv(scFv) molecule; (vi) dAb fragment.

In the fourth aspect of the present invention, it provides a recombinant protein, wherein the recombinant protein comprises:
(i) the nanobody targeting HER2 of the first aspect of the present invention, or the antibody targeting HER2 of the second aspect of the present invention; and
(ii) an optional polypeptide molecule or fragment with therapeutic function;
(iii) an optional functional domain that enhances protein physicochemical properties or drug properties.

In another preferred embodiment, the enhancement of the physicochemical properties or drug production of the protein comprises prolonging the half-life of the HER2-targeting nanobody.

In another preferred embodiment, the recombinant protein also comprises: (iv) an optional tag sequence that assists expression and/or purification.

In another preferred embodiment, the tag sequence is selected from the group consisting of: 6His tag, GGGS sequence, FLAG tag.

In another preferred embodiment, the recombinant protein is a monomer, dimer, or multimer.

In another preferred embodiment, the peptide molecule or fragment with therapeutic functions includes but are not limited to: peptide molecules or fragments targeting EGFR, TGFβ, BCMA, B7H6, GUCY2C, DLL3, CD38, CD123, CD19, CD20, CD22, B7-H3, GPC3, CD73, PMSA, CD28, 4-1BB, OX40, CD40, CD27, CD3, CTLA4, PD1, PDL1, BCMA, GLP-1, Trop2, TIG, LAG-3, FGL1, TLR7.

In another preferred embodiment, the peptide molecule or fragment with therapeutic functions includes but are not limited to: insulin, IL-2, interferon, calcitonin, GHRH peptide, intestinal peptide analogs, albumin, antibody fragments, cytokines.

In another preferred embodiment, the recombinant protein (or polypeptide) comprises a fusion protein.

In another preferred embodiment, the fusion protein comprises a multispecific antibody and a chimeric antibody.

In another preferred embodiment, the functional domain that enhances protein physicochemical properties or drug resistance includes Fc segment, albumin binding domain (ABD), and anti albumin nanobody (HLE).

In another preferred embodiment, the fusion protein has the following elements from the N-C end:
A-B or A-Linker-B;
wherein, the element A is a nanobody targeting HER2; the element B is Fc segment, albumin binding domain (ABD), or anti albumin nanobody (HLE);
"-" represents a peptide bond, Linker represents linker.

In another preferred embodiment, the VHH chain of the nanobody targeting HER2 is selected from the following group: the amino acid sequence shown in SEQ ID NO.4, SEQ ID NO.8, SEQ ID NO.12, SEQ ID NO.16, SEQ ID NO.20, or SEQ ID NO.24.

In another preferred embodiment, the Fc segment is human IgG Fc segment, preferably from human IgG1, or human IgG2.

In another preferred embodiment, the Fc segment includes either FC1 or FCWT.

In another preferred embodiment, the sequence of FC1 is shown as positions 126-357 of SEQ ID NO. 25, and the sequence of FCWT is shown as positions 126-357 of SEQ ID NO. 26.

In another preferred embodiment, the Linker is (G4S)n, where n is a positive integer from 1 to 4; Furthermore, the Linker may include modifications of one or more cysteine residues.

In another preferred embodiment, the fusion protein is 1-G07-FC1, 1-G07-FCWT, or 1-G07-ABD.

In another preferred embodiment, the amino acid sequence of 1-G07-FC1 is shown in SEQ ID NO.25.

In another preferred embodiment, the amino acid sequence of 1-G07-FCWT is shown in SEQ ID NO.26.

In another preferred embodiment, the amino acid sequence of 1-G07-ABD is shown in SEQ ID NO.27.

In the fifth aspect of the present invention, it provides a CAR construct, wherein the antigen binding region of the CAR construct is the VHH chain of the nanobody of the first aspect of the present invention.

In the sixth aspect of the invention, it provides a recombinant immune cell expressing exogenous CAR construct of the fifth aspect of the present invention.

In another preferred embodiment, the immune cell is selected from the group consisting of: a NK cell and a T cell.

In another preferred embodiment, the immune cell is derived from human or non-human mammals (such as mice).

In the seventh aspect of the present invention, it provides an immunoconjugate containing:
(a) an antibody moiety, wherein the antibody moiety is the nanobody targeting HER2 of the first aspect of the present invention, or the antibody targeting HER2 of the second aspect of the present invention, or the recombinant protein of the fourth aspect of the present invention; and
(b) a coupling moiety coupled to the nanobody moiety, which is selected from the group consisting of: a detectable label, a drug, a toxin, a cytokine, an enzyme, a protein degrading agent, an oligonucleoxylic acid, and a combination thereof.

In another preferred embodiment, the immunoconjugate is nanobody-drug conjugate.

In another preferred embodiment, the nanobody moiety is coupled to the coupling moiety through a chemical bond or linker.

In another preferred embodiment, the coupling moiety is chemical marker and biomarker.

In another preferred embodiment, the chemical marker is an isotope, an immunotoxin, and/or a chemical drug.

In another preferred embodiment, the biomarker is a biotin, avidin, or enzyme marker. In another preferred embodiment, the coupling moiety is a drug or toxin.

In another preferred embodiment, the drug is a cytotoxic drug.

In another preferred embodiment, the cytotoxic drug is selected from the group consisting of: an antitubulin drug, DNA sulcus binding reagent, DNA replication inhibitor, alkylation reagent, antibiotic, folic acid antagonist, antimetabolite, chemotherapeutic sensitizer, topoisomerase inhibitor, vinca alkaloids, and a combination thereof.

The particularly useful cytotoxic drug comprises, for example, DNA sulcus binding reagent, DNA alkylation reagent, and tubulin inhibitor. The typical cytotoxic drug includes, such as auristatins, camptothecins, duocarmycins, etoposides, maytansines and maytansinoids (such as DM1 and DM4), taxanes, benzodiazepines, or benzodiazepines containing drugs (for example pyrrolo [1,4] benzodiazepine (PBD), indolinobenzodiazepines, and oxazolidinobenzodiazepines, vinca alkaloids, and a combination thereof.

In another preferred embodiment, the toxin is selected from the group consisting of: otostatins (e.g., otostatin E, otostatin F, MMAE and MMAF), aureomycin, metametanol, ricin toxin, ricin A-chain, cobustatin, docamicin, dorastatin, doxorubicin, daunorubicin, paclitaxel, cisplatin, cc1065, ethidium bromide, mitomycin, etoposide, tenoposide, vincristine, vinblastine, colchicine, dihydroxy anthrax dione, actinomycin, diphtheria toxin, pseudomonas ectotoxin (PE)A, PE40, Acacia bean toxin, Acacia bean toxin A chain, capsule root toxin A chain, α-toesin, Atractylodes toxin, mitogellin, retstrictocin, phenomycin, enomycin, curicin, battocin, Kazinomycin, Sapaonaria officinalis inhibitor, glucocorticoids, and a combination thereof.

In another preferred embodiment, the coupling moiety is a detectable label.

In another preferred embodiment, the detectable label comprises a radionuclide, wherein the radionuclide comprises:
(i) a diagnostic isotope which is selected from the group consisting of Tc-99m, Ga-68, F-18, I-123, I-125, I-131, In-111, Ga-67, Cu-64, Zr-89, C-11, Lu-177, Re-188, and a combination thereof; and/or
(ii) a therapeutic isotope which is selected from the group consisting of Lu-177, Y-90, Ac-225, As-211, Bi-212, Bi-213, Cs-137, Cr-51, Co-60, Dy-165, Er-169, Fm-255, Au-198, Ho-166, I-125, 1-131, Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133, Yb-169, Yb-177, and a combination thereof.

In another preferred embodiment, the protein degrading agent is a degrading agent for tumor associated proteins selected from the group consisting of: EGFR, NF-κB, RIPK2, BCR-ABL, HER2, c-Met, TBK1, CDK, ALK, Akt, CK2, ERK 1/2, FLT3, PI3K, BTK, TRK, Fak, BRD, AR, ER, MetAp-2, BCL-XL, Sirt2, HDAC6, Pirin, SMAD3, ARNT, PCAF/GCN5, Tau, EZH2, IRAK4, STAT3 FRS2, RAS (such as KRAS, HRAS, and NRAS).

In another preferred embodiment, the KRAS comprises KRAS-G12C, KRAS-G12D, KRAS-G12V, etc.

In another preferred embodiment, the protein degrading agent comprises PROTAC (protein degradation targeted chimeras).

In another preferred embodiment, the PROTAC targeting EGFR, KRAS (comprises KRAS-G12C, KRAS-G12D, KRAS-G12V, etc).

In another preferred embodiment, the oligonucleotide comprises antisense oligonucleotide (ASO), small interfering RNA (siRNA), microRNA (miRNA), small activating RNA (saRNA), messenger RNA (mRNA), or RNA aptamer.

In another preferred embodiment, the coupling moiety is selected the group consisting of a fluorescent or luminescent marker, radioactive marker, MRI (magnetic resonance imaging) or CT (electronic computer X-ray tomography) contrast agent, or enzyme capable of producing a detectable product, radionuclide, biotoxin, cytokine (such as IL-2, etc.), antibody, antibody Fc fragment, antibody scFv fragment, gold nanoparticle/nanorod, virus particle, liposome, nanomagnetic particle, prodrug activating enzyme (e.g., DT-diaphorase (DTD) or biphenyl hydrolase-like protein (BPHL)), chemotherapeutic agent (e.g., cisplatin), or nanoparticle in any form.

In another preferred embodiment, the nanobody-drug conjugate contains: a nanobody targeting HER2 of the first aspect of the present invention, or the antibody targeting HER2 of the second aspect of the present invention with multivalency (e.g., bivalency).

In another preferred embodiment, the multivalency means that the amino acid sequence of the nanobody-drug conjugate contains multiple repeats of nanobody targeting HER2 of the first aspect of the present invention, or the antibody targeting HER2 of the second aspect of the present invention.

In another preferred embodiment, the test is in vivo or in vitro.

In another preferred embodiment, the nanobody-drug conjugate is used for diagnosing and/or treating tumors expressing HER2 protein.

In another preferred embodiment, the antibody-drug conjugate ADC is as shown in the following molecular formula: 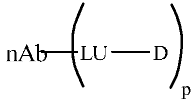 wherein,
nAb is a nanobody targeting HER2,
LU is a linker (also called connector);
D is a drug;
and the subscript p is a value selected from 1-8.

In another preferred embodiment, the LU is selected from the group consisting of: maleimidocaproyl (MC), maleimide (MAL), succinimidyl 4-(n-maleimidyl methyl) cyclohexane-1-formate) (SMCC) linker to partially connect with the antibody, and comprises one or more of the linkers of valine-citrulline (VC), valine-alanine (VA), glycine-glycine-phenylalanine-glycine (GGFG), alanine-alanine-alanine (AAA), p-aminobenzyloxycarbonyl (PAB), polyethylene glycol (PEG).

In another preferred embodiment, wherein the antibody is covalently bound to the linker by reacting with a moiety selected from the group consisting of: maleimidyl hexanoyl (MC), maleimide (MAL), succinimidyl 4-(n-maleimidyl methyl) cyclohexane-1-formate) (SMCC), etc.

In another preferred embodiment, the D is a compound with antitumor activity selected from the group consisting of:
(i) tubulin inhibitors, such as maytanin derivatives (DM1, DM4), monomethyl orrestatin E (MMAE), monomethyl orrestatin F (MMAF);
(ii) toxins that act on DNA, such as duocarmycin, pyrrolobenzodiazepine (PBD);
(iii) topoisomerase inhibitors, camptothecin, SN38, Exatecan, Dxd.

In another preferred embodiment, the LU-D compound is selected from the consisting of:

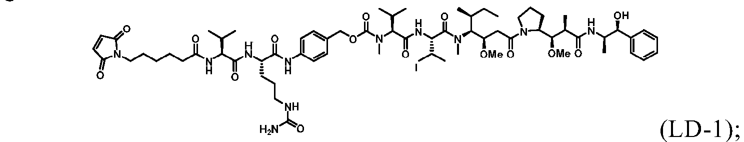

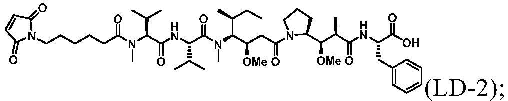

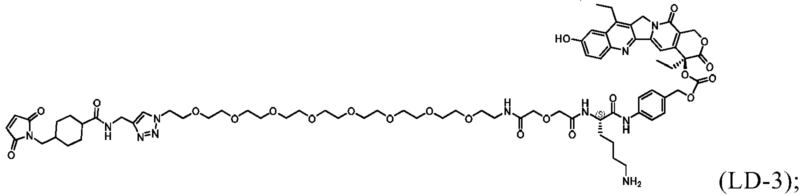

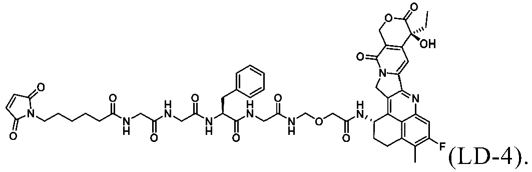

In the eighth aspect of the invention, it provides a pharmaceutical composition comprising:
(i) the nanobody targeting HER2 of the first aspect of the present invention, or the antibody targeting HER2 of the second aspect of the present invention, or the multispecific antibody of the third aspect of the present invention, or the recombinant protein of the fourth aspect of the present invention, or the recombinant immune cell of the sixth aspect of the present invention, or the nanobody-drug conjugate of the seventh aspect of the present invention;
(ii) a pharmaceutically acceptable carrier.

In another preferred embodiment, the pharmaceutical composition includes single drugs, compound drugs, or synergistic drugs.

In another preferred embodiment, the pharmaceutical composition also includes other biologically active drugs, such as those used to treat tumors.

In another preferred embodiment, the administration method of the pharmaceutical composition is selected from the group consisting of: subcutaneous injection, intradermal injection, intramuscular injection, intravenous injection, intraperitoneal injection, microneedle injection, oral administration, or oral and nasal spray and atomization inhalation.

In another preferred embodiment, the dosage form of the pharmaceutical composition is selected from the group consisting of liquid, solid, or gel.

In another preferred embodiment, the pharmaceutical composition is a liquid preparation.

In another preferred embodiment, the pharmaceutical composition is an injection.

In the ninth aspect of the present invention, it provides a use of an active ingredient selected from the group consisting of: the nanobody targeting HER2 of the first aspect of the present invention, or the antibody targeting HER2 of the second aspect of the present invention, or the multispecific antibody of the third aspect of the present invention, or the recombinant protein of the fourth aspect of the present invention, or the recombinant immune cell of the sixth aspect of the present invention, or the nanobody-drug conjugate of the seventh aspect of the present invention, and combinations thereof, wherein the active ingredient is used for (a) preparation of a detection reagent, a detection plate or a kit; and/or (b) preparing a drug for the prevention and/or treatment of a HER2-related disease.

In another preferred embodiment, the detection reagent, detection plate or kit is used for:
(1) detecting HER2 protein in a sample; and/or
(2) detecting endogenous HER2 protein in tumor cells; and/or
(3) detecting tumor cells expressing HER2 protein.

In another preferred embodiment, the detection reagent, detection plate or kit is used for diagnosing a HER2-related disease.

In another preferred embodiment, the drug is used for treating or preventing a HER2 high expression tumor, tumor migration, or tumor resistance.

In another preferred embodiment, the detection type includes but is not limited to flow cytometry, cell immunofluorescence detection, enzyme-linked immunosorbent assay, immunoblotting detection, etc.

In another preferred embodiment, the detection reagent, detection plate or kit is used for diagnosing HER2 related diseases.

In another preferred embodiment, the drug is used for treating or preventing HER2 overexpressing tumors, tumor migration, or tumor resistance.

In another preferred embodiment, the tumor resistance comprises: resistance of tumor immunotherapy drug, resistance of tumor targeted therapy drug, resistance of conventional tumor chemotherapy, and insensitivity to radiotherapy.

In another preferred embodiment, the drug is used for a use selected from the group consisting of:
(a) inhibiting HER2 kinase phosphorylation (activation);
(b) specifically binding to tumor cells, and/or HER2 of the immune/stromal cells in the tumor microenvironment;
(c) inhibiting tumor cell migration or metastasis;
(d) inhibiting tumor growth and improving the anti-tumor efficacy of combination drug therapy;
(e) promoting the proliferation, survival and function of immune cells, thereby improving the effect of tumor immunity.

In another preferred embodiment, the HER2-related disease is selected from the group consisting of cancer, an autoimmune disease, a metabolism-related disease, an infectious disease, and a combination thereof.

In another preferred embodiment, the HER2-related disease comprises: tumorigenesis, tumor growth and/or metastasis.

In another preferred embodiment, the cancer comprises a solid tumor and a hematologic cancer.

In another preferred embodiment, the cancer is a tumor with high HER2 expression.

In another preferred embodiment, the tumor with high HER2 expression is selected from the group consisting of breast cancer, gastric cancer, esophageal cancer, lung cancer, pancreatic cancer, ovarian cancer, prostate cancer, rectal cancer, glioma, melanoma, leukemia, lymphoma, and a combination thereof.

In another preferred embodiment, the cancer is a drug-resistant tumor.

In another preferred embodiment, the tumor with high HER2 expression refers to the ratio of the level L1 of HER2 transcript and/or protein in tumor tissue to the level L0 of HER2 transcript and/or protein in normal tissue, L1/L0 is ≥2, preferably ≥3.

In the tenth aspect of the present invention, it provides a polynucleotide encoding a polypeptide selected from the group consisting of:
(1) the nanobody targeting HER2 of the first aspect of the present invention, or the antibody targeting HER2 of the second aspect of the present invention, or the multispecific antibody of the third aspect of the present invention;
(2) the recombinant protein of the fourth aspect of the present invention; or
(3) the CAR construct of the fifth aspect of the present invention.

In another preferred embodiment, the polynucleotide comprises RNA, DNA or cDNA.

In the eleventh aspect of the present invention, it provides a vector comprising the polynucleotide of the tenth aspect of the present invention.

In another preferred embodiment, the vector comprises: a bacterial plasmid, a phage, a yeast plasmid, a plant cell virus, a mammalian cell virus such as an adenovirus, retrovirus, or other vectors.

In the twelfth aspect of the present invention, it provides a genetically engineered host cell comprising the vector of the eleventh aspect of the present invention or having the polynucleotide of the tenth present aspect of the invention integrated into its genome.

In the thirteenth aspect of the invention, it provides a method (including diagnostic or non-diagnostic method) for detecting HER2 in a sample *in vitro,* wherein the method comprising the steps:
(1) contacting a sample with the nanobody targeting HER2 of the first aspect of the present invention, the antibody targeting HER2 of the second aspect of the present invention, or the nanobody-drug conjugate of the seventh aspect of the present invention *in vitro;*
(2) detecting whether an antigen-antibody complex is formed, wherein the formation of a complex indicates the presence of HER2 in the sample.

In another preferred embodiment, the detecting comprises diagnostic or non-diagnostic method.

In the fourteenth aspect of the invention, it provides a detection plate comprising a substrate (or support plate) and a test strip, wherein the test strip comprising the nanobody targeting HER2 of the first aspect of the present invention, or the antibody targeting HER2 of the second aspect of the present invention, or the nanobody-drug conjugate of the seventh aspect of the present invention.

In the fifteenth aspect of the present invention, it provides a kit comprising:
(1) a first container containing the nanobody targeting HER2 of the first aspect of the present invention, or the antibody targeting HER2 of the second aspect of the present invention; and/or
(2) a second container containing a secondary antibody against the nanobody targeting HER2 of the first aspect of the present invention, or the antibody targeting HER2 of the second aspect of the present invention;
alternatively, the kit comprises the detection plate of the fourteenth aspect of the present invention.

In the sixteenth aspect of the present invention, it provides a method for preparing a recombinant polypeptide, which comprises the steps of:
(a) culturing the host cell of the twelfth aspect of the present invention under a condition suitable for expression;
(b) isolating a recombinant polypeptide from the culture, wherein the recombinant polypeptide is the nanobody targeting HER2 of the first aspect of the present invention, or the antibody targeting HER2 of the second aspect of the present invention, or the multispecific antibody of the third aspect of the present invention, or the recombinant protein of the fourth aspect of the present invention.

In the seventeenth aspect of the present invention, it provides a method for treating HER2-related diseases, wherein the method comprises: administering the nanobody targeting HER2 of the first aspect of the present invention, or the antibody targeting HER2 of the second aspect of the present invention, or the multispecific antibody of the third aspect of the present invention, or the recombinant protein of the fourth aspect of the present invention, or the recombinant immune cell of the sixth aspect of the present invention, or the nanobody-drug conjugate of the seventh aspect of the present invention, or the pharmaceutical composition of the eighth aspect of the present invention, and combinations thereof, to a subject in need.

In another preferred embodiment, the method further comprises: administering other drugs or treatment methods to the subject in need for a combined therapy.

In another preferred embodiment, the other drugs or treatment methods comprise: an anti-tumor immunotherapy drug, a tumor-targeted drug, a tumor chemotherapeutic agent, and tumor radiotherapy.

In another preferred embodiment, the anti-tumor immunotherapy drug comprises a PD-1, PD-L1, and EGFR monoclonal antibody.

In the eighteenth aspect of the invention, it provides a method for the preparation of a chimeric antibody, comprising the steps of:
cloning the nucleotide sequence of the alpaca-derived VHH sequence of the nanobody targeting HER2 of the first aspect of the present invention into an expression vector containing the nucleotide sequence of a human antibody constant region, and expressing the human-alpaca chimeric antibody by transfecting animal cells.

In the nineteenth aspect of the present invention, it provides a method for the preparation of a humanized antibody, comprising the steps of:
implanting the nucleotide sequences of the CDR regions in the VHH chain of the nanobody targeting HER2 of the first aspect of the present invention into a nucleoside sequence template containing human antibody FR regions, then cloning the resultant template into an expression vector containing the constant region of a human antibody, and expressing the humanized antibody by transfecting animal cells.

In the twentieth aspect of the invention, it provides a method for inhibiting tumor cell growth and migration, comprising the steps of: administering the nanobody targeting HER2 of the first aspect of the present invention, the antibody targeting HER2 of the second aspect of the present invention or the nanobody-drug conjugate of the present invention, or CAR-T cell of the nanobody, and a combination thereof to a subject in need.

In the twenty-first aspect of the present invention, it provides a method for inhibiting tumor growth in a model animal, comprising the steps of: administering the nanobody targeting HER2 of the first aspect of the present invention, the antibody targeting HER2 of the second aspect of the present invention, the nanobody -drug conjugate of the present invention, or CAR-T cell of the nanobody to a subject in need.

In another preferred embodiment, the drug can be administered alone or in combination with, such as, tumor immunotherapy, a tumor-targeted drug, a cytotoxic drug, and radiotherapy.

In the twenty-second aspect of the present invention, it provides a method for preparing the nanobody-drug conjugate of the seventh aspect of the present invention, comprising the steps:
(1) reacting an antibody with a reducing reagent in a buffer to obtain a reduced antibody;
(2) cross-linking (coupling) a linker-drug conjugate of formula DL with the reduced antibody obtained in step (1) in a mixture solution of a buffer solution and an organic solvent to obtain the antibody-drug conjugate 1a, 1b, 1c, or 1d with different DAR values.

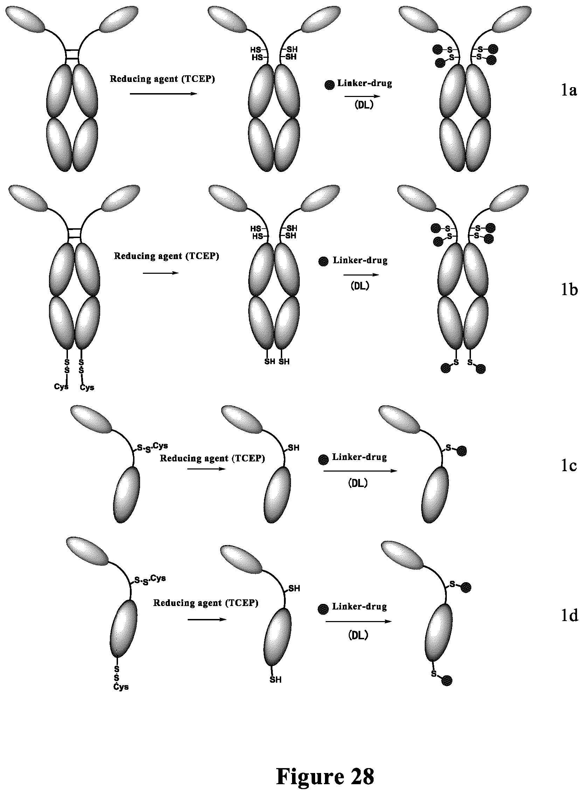

In another preferred embodiment, the cross-linking reaction of the preparation method is shown in FIG. 28.

In another preferred embodiment, in step (1), the antibody is reduced by a reducing reagent, so that the inter-chain disulfide bond in the antibody is reduced to produce sulfhydryl groups.

In another preferred embodiment, the reducing agent in step (1) is tris(2-carboxyethyl) phosphine hydrochloride (TCEP), beta-mercaptoethanol, beta-mercaptoethylamine hydrochloride, or dithiothreitol (DTT).

In another preferred embodiment, the buffer is selected from the group consisting of: potassium dihydrogen phosphate-sodium hydroxide (KH₂PO₄-NaOH)/sodium chloride (NaCl)/diethylene triamine pentacetate acid (DTPA) buffer, disodium hydrogen phosphate-citric acid/sodium chloride (NaCl)/diethylene triamine pentaacetic acid (DTPA), boric acid-borax/sodium chloride (NaCl)/diethylene triamine pentaacetic acid (DTPA), histidine-sodium hydroxide/sodium chloride (NaCl)/diethylene triamine pentaacetic acid (DTPA), and PBS/diethylene triamine pentaacetic acid (DTPA).

In another preferred embodiment, in step (2), the organic solvent in the reaction solution is no more than 15% by volume.

In another preferred embodiment, the organic solvent in step (2) is selected from the group consisting of: acetonitrile (ACN), dimethylformamide (DMF), dimethylacetamide (DMA), and dimethyl sulfoxide (DMSO).

Then the linker drug (10mg/ml, previously dissolved in acetonitrile (ACN), dimethylsulfoxide (DMSO), dimethylformamide (DMF) or diethylacetamide (DMA)) is added. It should be ensured that the volume ratio of the organic solvent in the reaction solution is no more than 15%. The coupling reaction is performed at 0-37 °C with stirring for 2-4 hours. If TCEP reduction is used, it is unnecessary to remove the remaining TCEP and the substituted maleimide compounds can be directly added for coupling.

The coupling reaction mixture is filtrated and purified by using a desalting column with sodium succinate/NaCl buffer or histidine-acetic acid/sucrose gel, and the peak samples are collected according to UV280 absorption value. Alternatively, ultrafiltration is performed for several times. After filtration and sterilization, the resultant product is stored at low temperature.

The drug/antibody coupling ratio (DAR) of the obtained antibody-drug conjugate is relatively uniform. For NDCs with certain differences in DAR, if a sample with better uniformity is needed, the following non-limitative methods can be further used for separation and purification: hydrophobic interaction chromatography (HIC), size-exclusion chromatography (SEC), ion exchange chromatography (IEC).

It is to be understood that the various technical features of the present invention mentioned above and the various technical features specifically described hereinafter (as in the Examples) may be combined with each other within the scope of the present invention to constitute a new or preferred technical solution, which needs not be described one by one, due to space limitations.

### DESCRIPTION OF DRAWINGS

Figure 1 shows the SDS-PAGE electrophoresis gel diagram after expression and purification of nine original HER2 nanobodies (HER2-VHH) and hIgG1-FC1 segment fusion proteins of the present invention.
Figure 2 shows the binding affinity EC₅₀ values of six HER2 VHH-FC1 fusion proteins 1-C10, 2-C07, 1-B05, 1-C09, 2-F03, 1-G07 to HER2-ECD.
Figure 3 shows the binding affinity constant (KD) of HER2 VHH-FC1 fusion proteins 1-C10, 2-C07, 1-B05, 1-C09, 2-F03 to human HER2-ECD determined by surface plasmon resonance (SPR) analysis.
Figure 4 shows the binding affinity of HER2 VHH-FC1 fusion protein to HER2 receptor on the surface of NCI-N87 cells, and the results show the binding curve and EC₅₀ value.
Figure 5 shows the inhibitory effect of HER2 VHH-FC1 antibody fusion protein on the growth of NCI-N87 xenograft tumors in nude mice.
Figure 6 shows the staining analysis of immunohistochemical staining of the tumor samples described in Figure 5 to detect the inhibitory activity of HER2 VHH-FC1 fusion protein on phosphorylated AKT after administration.
Figure 7 shows the SDS-PAGE electrophoresis gel images of 1-G07-FCWT and 1-G07-ABD after expression purification.
Figure 8 shows the hydrophobic interaction chromatographic profile (HIC) of the 1-G07-FC1 coupled VC-MMAE conjugate NDC.
Figure 9 shows the hydrophobic interaction chromatographic profile (HIC) of the 1-G07-ABD-coupled VC-MMAE conjugate NDC.
Figure 10 shows the hydrophobic interaction chromatographic profile (HIC) of the 2-C07-FC1 coupled VC-MMAE conjugate NDC.
Figure 11 shows the hydrophobic interaction chromatographic profile (HIC) of the 1-C10-FC1 coupled VC-MMAE conjugate NDC.
Figure 12 shows the hydrophobic interaction chromatographic profile (HIC) of the 1-C09-FC1 coupled VC-MMAE conjugate NDC.
Figure 13 shows the hydrophobic interaction chromatographic profile (HIC) of the 1-B05-FC1 coupled VC-MMAE conjugate NDC.
Figure 14 shows the hydrophobic interaction chromatographic profile (HIC) of the 2-F03-FC1 coupled VC-MMAE conjugate NDC.
Figure 15 shows the hydrophobic interaction chromatographic profile (HIC) of the 1-G07-FC1 coupled GGFG-DXd conjugate NDC.
Figure 16 shows the inhibitory activity of six HER2-NDCs against the proliferation of N87 cells, showing the inhibition curve and IC₅₀ value.
Figure 17 shows the inhibitory activity of six HER2-NDCs against SK-BR-3 cell proliferation, showing the inhibition curve and IC₅₀ value.
Figure 18 shows the inhibitory activity of six HER2-NDCs against BT474 cell proliferation, showing the inhibition curve and IC₅₀ value.
Figure 19 shows the inhibitory activities of 1-G07-FCWT-MMAE, 1-G07-FC1-MMAE, and 1-G07-ABD-MMAE against the proliferation of HCC1954 cells, showing the inhibition curves and IC₅₀ values.
Figure 20 shows the inhibitory activities of 1-G07-FCWT-MMAE, 1-G07-FC1-MMAE, and 1-G07-ABD-MMAE against the proliferation of N87 cells, showing the inhibition curves and IC₅₀ values.
Figure 21 shows the inhibitory activity of 1-G07-FC1-DXd against the proliferation of N87 and HCC1954 cells, showing the inhibition curve and IC₅₀ value.
Figure 22 shows the therapeutic effects of 1-C09-FC1-MMAE, 1-B05-FC1-MMAE and 2-F03-FC1-MMAE on N87 xenograft tumor model in nude mice after a single intravenous injection of 3mg/kg, showing the tumor growth curve and tumor inhibition rate statistical data.
Figure 23 shows the therapeutic effects of 2-C07-FC1-MMAE, 1-C10-FC1-MMAE and 1-G07-FC1-MMAE on N87 xenograft tumor model in nude mice after a single intravenous injection of 3mg/kg, showing the tumor growth curve and tumor inhibition rate statistical data.
Figure 24 shows the therapeutic effects of 2-C07-FC1-MMAE, 1-C10-FC1-MMAE and 1-G07-FC1-MMAE on N87 xenograft tumor model in nude mice after a single intravenous injection of 1mg/kg or 0.3mg/kg, showing the tumor growth curve and tumor inhibition rate statistical data.
Figure 25 shows the therapeutic effects of 1-G07-FCWT-MMAE and 1-G07-ABD-MMAE on large-volume N87 tumor regression experiment in nude mice after a single intravenous injection of 3mg/kg or 10 mg/kg of T-DXd, showing the tumor growth curve and tumor inhibition rate statistical data.
Figure 26 shows the therapeutic effect of 1-G07-FC1-DXd on large-volume N87 tumor regression experiment in nude mice after intravenous injection of 20mg/kg once a week for a total of 2 weeks, showing the tumor growth curve and tumor inhibition rate statistical data.
FIG. 27 shows the structural formula of the fusion protein designed for the present invention.
FIG. 28 shows the cross-linking reaction formula for the preparation method of the nanobody-drug conjugate of the present invention, wherein antibody-drug conjugates with different DAR values are shown as 1a, 1b, 1c, or 1d, respectively.

### Modes for Carrying Out the Present Invention

Through extensive and in-depth research, the inventor established a yeast display library by immunizing Alpaca, and obtained 1-C10, 2-C07, 1-B05, 1-C09, 2-F03, 1-G07 and other nano-antibodies after mass screening. The antibodies can bind human HER2 protein with high specificity. The EC₅₀ of the antibodies determined by ELISA was 0.017µg/mL, 0.005µg/mL, 0.005µg/mL, 0.01µg/mL, 0.004µg/mL, 0.01µg/mL, respectively. The binding constants determined by surface plasmon oscillation (SPR) were 4.613nM, 7.418nM, 8.896nM, 12.06nM and 23.24nM, respectively. The dual antibody, multifunctional fusion protein and antibody-drug conjugate based on nanobody are expected to not only retain the same effects as antibodies, but also provide convenient functional combination of anti-tumor activity and improve the therapeutic effect.

### Terms

As used herein, the terms "nanobody of the present invention", "anti-HER2 nanobody of the present invention", "HER2 nanobody of the present invention", "ErbB2/HER2-targeting nanobody of the present invention (HER2-Nb)" are used interchangeably, all referring to nanobodies that specifically recognize and bind to HER2 (including human HER2).

As used herein, the term "antibody" or "immunoglobulin" is a heterotetrameric glycoprotein of about 150,000 Da having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by a plurality of constant regions. Each light chain has a variable region (VL) at one end and a constant region at the other end; the constant region of a light chain pairs with the first constant region of a heavy chain, and the variable region of a light chain pairs with the variable region of a heavy chain. Special amino acid residues form an interface between the variable regions of a light chain and a heavy chain.

As used herein, the terms "single domain antibody (VHH)" and "nanobody" have the same meaning and refer to the variable region of the heavy chain of the monoclonal antibody, constructing a nanobody (VHH) composed of only one heavy chain variable region, which is the smallest antigen-binding fragment with complete function. Usually, the antibody with natural deletion of light chain and heavy chain constant region 1(CH1) is obtained first, and then the variable region of the antibody heavy chain is cloned to construct a nanobody (VHH) composed of only one heavy chain variable region.

As used herein, the term "variable" means that antibodies are different from each other in terms of sequence in certain parts of variable regions, which is responsible for the binding and specificity of various specific antibodies to their specific antigens. However, the variability is not distributed evenly throughout the variable regions of an antibody. It is concentrated in three segments called complementarity determining regions (CDRs) or hypervariable regions in the light and heavy chain variable regions. The conserved parts of variable regions are called framework regions (FRs). Each of the variable regions of naturally occurring heavy and light chains comprises four FR regions, which are generally in a β-sheet configuration, joined by the three CDRs forming a linking loop, and in some cases, may form a partial β-sheet structure. The CDRs in each chain are closely linked together via the FR regions, and together with the CDRs of the other chain, form the antigen binding site of an antibody (see Kabat et al., NIH Publ. No. 91-3242, Vol. I, pp. 647-669 (1991)). The constant regions are not directly involved in the binding of an antibody to an antigen, however, they exhibit different effector functions, for example, and they are involved in the antibody-dependent cytotoxicity of an antibody.

As known by those skilled in the art, immunoconjugates and fusion expression products include: conjugates formed by binding drugs, toxins, cytokines, radionuclides, enzymes, and other diagnostic or therapeutic molecules to the nanobodies or fragments thereof of the present invention. The invention also includes a cell surface marker or an antigen that binds to said anti-HER2 protein nanobody or the fragment thereof.

As used herein, the term "heavy chain variable region" and "VH" can be used interchangeably.

As used herein, the terms "variable region" and "complementary determining region (CDR)" can be used interchangeably.

In another preferred embodiment, the heavy chain variable region of said nanobody comprises 3 complementary determining regions: CDR1, CDR2, and CDR3.

In another preferred embodiment, the heavy chain of said nanobody comprises the above said heavy chain variable region and a heavy chain constant region.

According to the present invention, the terms "nanobody of the invention", "protein of the invention", and "polypeptide of the invention" are used interchangeably and all refer to a polypeptide, such as a protein or polypeptide having a heavy chain variable region, that specifically binds to HER2 protein. They may or may not contain a starting methionine.

The invention also provides other proteins or fusion expression products having the nanobodies of the invention. Specifically, the present invention includes any protein or protein conjugate and fusion expression product (i.e. immunoconjugate and fusion expression product) having a heavy chain containing a variable region, as long as the variable region are identical or at least 90% identical, preferably at least 95% identical to the heavy chain of the nanobody of the present invention.

In general, the antigen-binding properties of a nanobody can be described by three specific regions located in the variable region of the heavy chain, referred as variable regions (CDRs), and the segment is divided into four frame regions (FRs). The amino acid sequences of four FRs are relatively conservative and do not directly participate in binding reactions. These CDRs form a loop structure in which the β-sheets formed by the FRs therebetween are spatially close to each other, and the CDRs on the heavy chain and the CDRs on the corresponding light chain constitute the antigen-binding site of the nanobody. The amino acid sequences of the same type of nanobodies can be compared to determine which amino acids constitute the FR or CDR regions.

The variable regions of the heavy chains of the nanobodies of the invention become a particular interest because at least a part of them is involved in binding antigens. Thus, the present invention includes those molecules having a nanobody heavy chain variable region with a CDR, provided that their CDRs are 90% or more (preferably 95% or more, the most preferably 98% or more) identical to the CDRs identified herein.

### Anti-HER2 nanobody

The present invention provides a variety of HER2-targeting nanobodies with high specificity and high affinity comprising only a heavy chain containing a heavy chain variable region (VH) amino acid sequence (listed in Table-1 and Table-2).

Preferably, the amino acid sequence of heavy chain variable region (VH) comprises CDR1, CDR2 and CDR3 having the following polypeptide sequences:
a1) CDR1 is SEQ ID NO.1: GLTFRRYE, SEQ ID NO.5: GLTFMRYD, SEQ ID NO.9: GITFFRHT, SEQ ID NO.13: GLTFRRYH, SEQ ID NO.17: GLTFRRYH, SEQ ID NO.21: RITFRHYD;
a2) CDR2 is SEQ ID NO.2: ITPASGSR, SEQ ID NO.6: LDSSSGRT, SEQ ID NO.10: ITGGESTN, SEQ ID NO.14: ISDTGSTT, SEQ ID NO.18: ISDTGTTN, SEQ ID NO.22: VTNGGVIT;
a3) CDR3 is SEQ ID NO.3: NAYWMIPQHIENNY, SEQ ID NO.7: NAQWKRIGADY, SEQ ID NO.11: NSKWDAAGVEF, SEQ ID NO.15: NAKWPIIAADW, SEQ ID NO.19: NGRWPAVAADW, SEQ ID NO.23: NAVWVYLQRLTQNKKENDY;
a4) a sequence with HER2 binding affinity which is obtained through addition, deletion, modification and/or substitution of at least one amino acid of any amino acid sequence of the above amino acid sequences.

In another preferred embodiment, the sequence obtained through addition, deletion, modification and/or substitution of at least one amino acid is preferably an amino acid sequence having a homology of at least 80%, preferably at least 85%, more preferably at least 90%, and most preferably at least 95%.

Preferably, the antibody can inhibit the catalytic function of HER2 on the cell surface and recombinant HER2, and the antibody can be quickly internalized into intracellular lysosome.

The antibody of the present invention may be a double-chain or single-chain antibody, and may be selected from an animal-derived antibody, a chimeric antibody, a human-animal chimeric antibody, and preferably is a humanized antibody, and more preferably a fully humanized antibody.

The antibody derivative of the present invention may be a single-chain antibody, and/or an antibody fragment, for example, Fab, Fab', (Fab')₂ or other antibody derivatives known in the art, etc., and may be any one or more of IgA, IgD, IgE, IgG and IgM antibodies or other subtype antibodies.

In the present invention, the animal is preferably a mammal, such as mouse.

The antibody of the present invention may be a chimeric antibody, a humanized antibody, a CDR grafted and/or modified antibody that targets human HER2.

In a preferred embodiment of the present invention, any one or more sequences of SEQ ID NOs. 1-3, SEQ ID NOs. 5-7, SEQ ID NOs. 9-11, NOs. 13-15, NOs. 17-19, and SEQ ID NOs. 21-23 or sequences thereof that are obtained through addition, deletion, modification and/or substitution of at least one amino acid and have HER2 binding affinity, are located in the CDRs of heavy chain variable region (VH).

In above content of the present invention, the number of the added, deleted, modified and/or substituted amino acids, preferably does not exceed 40%, more preferably does not exceed 35%, is more preferably 1-33%, is more preferably 5-30%, is more preferably 10-25%, and is more preferably 15-20% of the total number of the amino acids of the initial amino acid sequence.

In the above content of the present invention, more preferably, the number of the added, deleted, modified and/or substituted amino acids, may be 1-7, more preferably 1-5, more preferably 1-3, and more preferably 1-2.

In another preferred embodiment, the antibody is human-alpaca chimeric antibody 1-C10, 2-C07, 1-B05, 1-C09, 2-F03, 1-G07.

In another preferred embodiment, the amino acid sequence ID numbers of the heavy chain variable regions (VH) of the chimeric antibody is listed in **Table-1.**

In another preferred embodiment, the amino acid sequence ID numbers of the complementarity determining region (CDR) of the antibody is listed in **Table-2.**

The antibody of the present invention can be used in combination, for constructing CAR constructs, recombinant immune cells containing CAR constructs, antibody drug conjugates, etc., and can also be used for (a) preparation of a detection reagent, detection plate or kit; and/or (b) preparation of a medicine for preventing and/or treating a HER2-related disease.

**Table-1: The representative meaning of each sequence in the sequence listing of the present invention**

| Sequence number | Sequence name | Sequence number | Sequence name |
|---|---|---|---|
| SEQ ID NO.1 | 1-C10 CDR1 | SEQ ID NO.15 | 1-C09 CDR3 |
| SEQ ID NO.2 | 1-C10 CDR2 | SEQ ID NO.16 | 1-C09 VH |
| SEQ ID NO.3 | 1-C10 CDR3 | SEQ ID NO.17 | 2-F03 CDR1 |
| SEQ ID NO.4 | 1-C10 VH | SEQ ID NO.18 | 2-F03 CDR2 |
| SEQ ID NO.5 | 2-C07 CDR1 | SEQ ID NO.19 | 2-F03 CDR3 |
| SEQ ID NO.6 | 2-C07 CDR2 | SEQ ID NO.20 | 2-F03 VH |
| SEQ ID NO.7 | 2-C07 CDR3 | SEQ ID NO.21 | 1-G07 CDR1 |
| SEQ ID NO.8 | 2-C07 VH | SEQ ID NO.22 | 1-G07 CDR2 |
| SEQ ID NO.9 | 1-B05 CDR1 | SEQ ID NO.23 | 1-G07 CDR3 |
| SEQ ID NO.10 | 1-B05 CDR2 | SEQ ID NO.24 | 1-G07 VH |
| SEQ ID NO.11 | 1-B05 CDR3 | SEQ ID NO.25 | 1-G07-FC1 |
| SEQ ID NO.12 | 1-B05 VH | SEQ ID NO.26 | 1-G07-FCWT |
| SEQ ID NO.13 | 1-C09 CDR1 | SEQ ID NO.27 | 1-G07-ABD |
| SEQ ID NO.14 | 1-C09 CDR2 | | |

**Table-2: CDR amino acid sequence of HER2 nanobody of the present invention**

| | CDR1 | CDR2 | CDR3 |
|---|---|---|---|
| 1-C10 | GLTFRRYE (SEQ ID NO.1) | ITPASGSR (SEQ ID NO.2) | NAYWMIPQHIENNY (SEQ ID NO.3) |
| 2-C07 | GLTFMRYD (SEQ ID NO.5) | LDSSSGRT (SEQ ID NO.6) | NAQWKRIGADY (SEQ ID NO.7) |
| 1-B05 | GITFFRHT (SEQ ID NO.9) | ITGGESTN (SEQ ID NO.10) | NSKWDAAGVEF (SEQ ID NO.11) |
| 1-C09 | GLTFRRYH (SEQ ID NO.13) | ISDTGSTT (SEQ ID NO.14) | NAKWPIIAADW (SEQ ID NO.15) |
| 2-F03 | GLTFRRYH (SEQ ID NO.17) | ISDTGTTN (SEQ ID NO.18) | NGRWPAVAADW (SEQ ID NO.19) |
| 1-G07 | RITFRHYD (SEQ ID NO.21) | VTNGGVIT (SEQ ID NO.22) | NAVWVYLQRLTQNKKENDY (SEQ ID NO.23) |

### Recombinant protein (or fusion protein)

In the present invention, it also includes recombinant proteins (or fusion proteins) containing HER2 nanobodies of the present invention. A preferred fusion protein is a multispecific antibody, which further comprises a second antigen binding region targeting a target selected from the group consisting of EGFR, TGF β, BCMA, B7H6, GUCY2C, DLL3, CD38, CD123, CD19, CD20, CD22, B7-H3, GPC3, CD73, PMSA, CD28, 4-1BB, OX40, CD40, CD27, CD3, CTLA4, PD1, PDL1, BCMA, GLP-1, Trop2, TIGIT, LAG-3, FGL1, TLR7, or combinations thereof.

Preferably, the multispecific antibody comprises one or more second antigen binding regions, or further comprises a third antigen binding region.

In addition, the recombinant protein (or fusion protein) of the present invention, in addition to containing the HER2 nanobody of the present invention, also includes optional tag sequences that assist in expression and/or purification (such as 6His tags, GGGS sequences, FLAG tags); or including optional peptide molecules or fragments with therapeutic functions; or optional protein functional domains that assist in physicochemical or pharmaceutical processes (such as molecules that can prolong the half-life of nanobodies in vivo, such as HLE, ABD).

In another preferred embodiment, the recombinant protein has the following elements from the N-C end:
A-B or A-Linker-B;
wherein, the element A is a nanobody targeting HER2; the element B is Fc segment, albumin binding domain (ABD), or anti albumin nanobody (HLE);
"-" represents a peptide bond, Linker represents linker.

It should be understood that the recombinant protein (or fusion protein) of the present invention may include a linker connecting the HER2 nanobody of the present invention and the protein functional domain.

Some residues (such as Cys or Lys) in the recombinant protein of the present invention are used to connect with multiple functional groups to achieve drug coupling. In a preferred embodiment, the B element or linker in the recombinant protein of the present invention can introduce a thiol group by introducing cysteine residues.

The present invention includes not only intact nanobodies but also fragment(s) of immunologically active nanobody or fusion protein(s) formed from nanobodies with other sequences. Therefore, the present invention also includes fragments, derivatives and analogs of the nanobodies.

As used herein, the terms "fragment", "derivative", and "analog" refer to a polypeptide that substantially retains the same biological function or activity of a nanobody of the invention. Polypeptide fragments, derivatives or analogs of the invention may be (i) polypeptides having one or more conservative or non-conservative amino acid residues (preferably conservative amino acid residues) substituted. Such substituted amino acid residues may or may not be encoded by the genetic code; or (ii) a polypeptide having a substituent group in one or more amino acid residues; or (iii) a polypeptide formed by fusing a mature polypeptide and another compound (such as a compound that increases the half-life of the polypeptide, for example, polyethylene glycol); or (iv) a polypeptide formed by fusing an additional amino acid sequence to the polypeptide sequence (e.g., a leader or secretory sequence or a sequence used to purify this polypeptide or a proprotein sequence, or a fusion protein formed with a 6 His tag). According to the teachings herein, these fragments, derivatives, and analogs are within the scope of one of ordinary skill in the art.

The nanobody of the present invention refers to a polypeptide including the above CDR regions having HER2 protein binding activity. The term also encompasses variant forms of polypeptides comprising the above CDR regions that have the same function as the nanobodies of the invention. These variations include, but are not limited to, deletions, insertions and/or substitutions of one or several (usually 1-50, preferably 1-30, more preferably 1-20, most preferably 1-10) amino acids, and addition of one or several (generally less than 20, preferably less than 10, and more preferably less than 5) amino acids at C-terminus and/or N-terminus. For example, in the art, the substitution of amino acids with analogical or similar properties usually does not alter the function of the protein. For another example, addition of one or several amino acids at the C-terminus and/or N-terminus usually does not change the function of the protein. The term also includes active fragments and active derivatives of the nanobodies of the invention.

The variant forms of the polypeptide include: homologous sequences, conservative variants, allelic variants, natural mutants, induced mutants, proteins encoded by DNAs capable of hybridizing with DNA encoding the nanobody of the present invention under high or low stringent conditions, and polypeptides or proteins obtained using antiserum against the nanobodies of the invention.

The invention also provides other polypeptides, such as a fusion protein comprising nanobodies or fragments thereof. In addition to almost full-length polypeptides, the present invention also includes fragments of the nanobodies of the invention. Typically, the fragment has at least about 50 contiguous amino acids of the nanobody of the invention, preferably at least about 50 contiguous amino acids, more preferably at least about 80 contiguous amino acids, and most preferably at least about 100 contiguous amino acids.

In the present invention, "a conservative variant of a nanobody of the present invention" refers to the polypeptides in which there are up to 10, preferably up to 8, more preferably up to 5, and most preferably up to 3 amino acids substituted by amino acids having analogical or similar properties, compared to the amino acid sequence of the nanobody of the present invention. These conservative variant polypeptides are preferably produced according to the amino acid substitutions in Table A.

**Table A**

| Original residue | Representative substitution | Preferable substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides a polynucleotide molecule encoding the above nanobody or fragment or fusion protein thereof. Polynucleotides of the invention may be in the form of DNA or RNA. DNA forms include cDNA, genomic DNA, or synthetic DNA. DNA can be single-stranded or double-stranded. DNA can be a coding strand or a non-coding strand.

Polynucleotides encoding the mature polypeptides of the invention include: coding sequences only encoding mature polypeptide; coding sequences for the mature polypeptide and various additional coding sequences; coding sequences (and optional additional coding sequences) and non-coding sequences for the mature polypeptide.

The term "polynucleotide encoding a polypeptide" may include a polynucleotide that encodes the polypeptide, and may also include a polynucleotide that includes additional coding and/or non-coding sequences.

The invention also relates to polynucleotides that hybridize to the sequences described above and that have at least 50%, preferably at least 70%, and more preferably at least 80% identity between the two sequences. The present invention specifically relates to polynucleotides that can be hybridized to the polynucleotides of the present invention under stringent conditions. In the present invention, "stringent conditions" refers to: (1) hybridization and elution at lower ionic strength and higher temperature, such as 0.2 x SSC, 0.1% SDS, 60°C; or (2) additional denaturants during hybridization, such as 50% (v/v) formamide, 0.1% fetal bovine serum / 0.1% Ficoll, 42°C, etc.; or (3) hybridization occurs only under the identity between the two sequences at least over 90%, preferably over 95%. Also, polypeptides encoded by hybridizable polynucleotides have the same biological functions and activities as mature polypeptides.

The full-length nucleotide sequence of the nanobody of the present invention or a fragment thereof can generally be obtained by a PCR amplification method, a recombination method, or an artificial synthesis method. One possible method is to synthesize related sequences using synthetic methods, especially when the fragment length is short. In general, a long sequence of fragments can be obtained by first synthesizing a plurality of small fragments and then connecting them. In addition, the coding sequence of the heavy chain and the expression tag (eg, 6His) can be fused together to form a fusion protein.

### Antibody preparation

The sequence of the DNA molecule for the antibody or a fragment thereof according to the present invention can be obtained by conventional techniques, for example, methods such as PCR amplification or genomic library screening. In addition, the sequences encoding light chain and heavy chain can be fused together, to form a single-chain antibody.

Once a relevant sequence is obtained, the relevant sequence can be obtained in bulk using a recombination method. This is usually carried out by cloning the sequence into a vector, transforming a cell with the vector, and then separating the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence.

At present, it is possible to obtain a DNA sequence encoding the antibody of the present invention (or fragments thereof, or derivatives thereof) completely by chemical synthesis. The DNA sequence can then be introduced into a variety of existing DNA molecules (or, for example, vectors) and cells known in the art. In addition, mutations can also be introduced into the protein sequences of the present invention by chemical synthesis.

The present invention further relates to a vector comprising said suitable DNA sequence and a suitable promoter or a control sequence. These vectors can be used to transform suitable host cells to enable them to express protein.

The host cell can be a prokaryotic cell, such as a bacterial cell; or a lower eukaryotic cell, such as a yeast cell; or a higher eukaryotic cell, such as a mammalian cell. Preferred animal cells include, but are not limited to, CHO-S, HEK-293 cells.

In general, under conditions suitable for expression of the antibody according to the present invention, the host cell obtained is cultured. Then, the antibody of the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained can be identified by conventional means. For example, the binding specificity of a monoclonal antibody can be determined by immunoprecipitation or an in vitro binding assay (such as radioimmunoassay (RIA) or enzyme-linked immunosorbent assay (ELISA)). The binding affinity of a monoclonal antibody can be determined by, for example, the Scatchard analysis (Munson et al., Anal. Biochem. 107: 220 (1980)).

The antibody according to the present invention can be expressed in a cell or on the cell membrane, or is secreted extracellularly. If necessary, the recombinant protein can be separated and purified by various separation methods according to its physical, chemical, and other properties. These methods are well known to those skilled in the art. The examples of these methods comprise, but are not limited to, conventional renaturation treatment, treatment by protein precipitant (such as salt precipitation), centrifugation, cell lysis by osmosis, ultrasonic treatment, super centrifugation, molecular sieve chromatography (gel chromatography), adsorption chromatography, ion exchange chromatography, high performance liquid chromatography (HPLC), and any other liquid chromatography, and a combination thereof.

### Nanobody-drug conjugate (NDC)

The present invention also provides an immunoconjugate based on an antibody of the present invention, preferably a nanobody-drug conjugate (NDC), and a method for preparing and use of an anti-HER2 nanobody-drug conjugate (HER2-NDC).

Typically, the antibody-drug conjugate comprises the antibody and an effector molecule, wherein the antibody is conjugated to the effector molecule, and chemical conjugation is preferred. Preferably, the effector molecule is a therapeutically active drug. In addition, the effector molecule may be one or more of a toxic protein, a chemotherapeutic drug, a small-molecule drug or a radionuclide.

The antibody according to present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that links an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. An antibody can be conjugated to a functional agent to form a conjugate of the antibody-functional agent. A functional agent (e.g. a drug, a detection reagent, a stabilizer) is conjugated (covalently linked) to an antibody. A functional agent can be linked to an antibody either directly or indirectly via a linker.

Nanobodies can be conjugated to drugs to form antibody-drug conjugates (NDCs). Typically, a NDC comprises a linker between a drug and an antibody. The linker can be a degradable or non-degradable linker. Typically, degradable linkers are easily degraded in an intracellular environment, for example, the linker is degraded at the target site, thereby releasing the drug from the antibody. Suitable degradable linkers include, for example, enzyme-degradable linkers, including peptidyl-containing linkers that can be degraded by protease (e.g. lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g. linkers that are hydrolyzed at a pH of below 5.5, such as hydrazone linkers) and linkers that are degraded under reducing conditions (e.g. disulfide-bond linkers). A non-degradable linker typically releases a drug under conditions that the antibody is hydrolyzed by protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g. iodo-, bromo- or chloro-substituted) amide; a halogenated (e.g. iodo-, bromo- or chloro-substituted) ester; a halogenated (e.g. iodo-, bromo- or chloro-substituted) methyl ketone, a benzyl halide (e.g. iodide, bromide or chloride); vinyl sulfone, pyridyl disulfide; a mercury derivative such as 3,6-di-(mercurymethyl)dioxane, wherein the counter ion is CH³COO⁻, Cl⁻ or NO³⁻; and polymethylene dimethyl sulfide thiosulfonate. The linker may include, for example, a maleimide linked to an antibody via thiosuccimide.

A drug may be any cytotoxic drug which inhibits cell growth or immunosuppression. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. Examples of particularly useful cytotoxic drugs include, for example, DNA minor groove binding agents, DNA alkylating agents, and tubulin inhibitors; typical cytotoxic drugs include, for example, auristatins, camptothecins, docamycin/duocarmycins, etoposides, maytansines and maytansinoids (e.g. DM1 and DM4), taxanes, benzodiazepines or benzodiazepine containing drugs (e.g. pyrrolo[1,4]benzodiazepines (PBDs), indolinobenzodiazepines and oxazolidinobenzodiazepines), and vinca alkaloids.

In the present invention, a drug-linker can be used to form a NDC in a simple step. In other embodiments, a bifunctional linker compound can be used to form a NDC in a two-step or multi-step process. For example, a cysteine residue is reacted with the reactive moiety of a linker in a first step, and then the functional group on the linker is reacted with a drug in the subsequent step, so as to form a NDC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in "Bioconjugation Technology" (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

Preferably, the coupling moiety of the nanobody drug conjugate NDC in the present invention that is coupled to the nanobody moiety comprises a protein degrading agent or an oligonucleotide drug.

Preferably, the oligonucleotide drug is a nucleic acid drug targeting small molecules, including antisense nucleic acid (ASO), small interfering RNA (siRNA), microRNA (miRNA), small activating RNA (saRNA), messenger RNA (mRNA), aptamer, ribozyme, antibody nucleic acid conjugate drug (ARC), etc.

As used herein, the protein degrading agent is a degrading agent for tumor associated proteins selected from the following group:
(1) Kinases such as RIPK2, BCR-ABL, EGFR, HER2, c-Met, TBK1, CDK2/4/6/9, ALK, Akt, CK2, ERK1/2, FLT3, PI3K, BTK, Fak, etc;
(2) BET proteins, such as BRD2/4/6/9;
(3) Nuclear receptors such as AR, ER, etc;
(4) Other proteins, such as MetAp-2, Bcl-xL, Sirt2, HDAC6, Pirin, SMAD3, ARNT, PCAF/GCN5, Tau, FRS2, RAS (KRAS, HRAS, and NRAS), etc.

Preferably, the protein degrading agent comprises PROTAC (Protein Degradation Targeting Chimera), and more preferably, the PROTAC targets EGFR, KRAS (e.g. KRAS-G12C, KRAS-G12D).

The present invention further provides a method for preparing a NDC, which may further comprise: under conditions sufficient to form an antibody-drug conjugate (NDC), binding an antibody to a drug-linker compound.

In certain embodiments, the method according to the present invention comprises: under conditions sufficient to form an antibody-linker conjugate, binding an antibody to a bifunctional linker compound. In these embodiments, the method according to the present invention further comprises: under conditions sufficient to covalently link the drug moiety to the antibody via a linker, binding the antibody-linker conjugate to the drug moiety.

In some embodiments, a nanobody-drug conjugate (NDC) has a formula as follows: 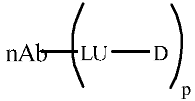 wherein:
nAb is a nanobody,
LU is a linker/connector;
D is a drug;
and the subscript p is a value selected from1 to 8.

### Drug

As used herein, the term "drug" refers to any compound possessing a desired biological activity and a reactive functional group available for preparing the conjugate of the invention. The desired biological activity includes activity useful in the diagnosis, cure, mitigation, treatment, or prevention of a disease in human or other animal. Thus, so long as it has the needed reactive functional group, the compound involved by the term "drug" include drugs identified in the official national pharmacopeia as well as e.g., official Homeopathic Pharmacopeia of the United States, or official National Formulary, or any supplements thereof. Exemplary drugs are set forth in the Physician's Desk Reference (PDR) and in the Orange Book maintained by the U.S. Food and Drug Administration (FDA). It should be understood that, as new drugs are continually discovered and developed, these drugs shall also be incorporated into the "drug" of the drug conjugates of the present invention.

The drugs useful to constitute the NDC of the present invention include, but are not limited to, cytotoxic agents (such as small molecule cytotoxic drugs).

The term "cytotoxic agents" refer to substances that inhibit or block cell expression activity, cell function and/or result in cell destruction. The term includes radioisotopes, chemotherapeutics, and toxins, such as small-molecular toxins or enzymatically active toxins (including fragments and/or variants thereof) derived from bacteria, fungi, plants or animals. Examples of cytotoxic agents include, but are not limited to: Auristatins (for example, Auristatin E, Auristatin F, MMAE and MMAF), chlortetracycline, metotanol, ricin, ricin A-chain, cobustatin, dokamicin, Dorastatin, adriamycin, daunorubicin, paclitaxel, cisplatin, cc1065, ethidium bromide, mitomycin, etoposide, tenoposide, vincristine, vinblastine, colchicine, dihydroxyanthracnose diketone, actinomycin, diphtheria toxin, Pseudomonas exotoxin (PE) A, PE40, abrin, abrin A chain, modeccin A chain, α-Sarcina, gelonin, mitogellin, retstrictocin, phenomycin, enomycin, curicin, crocotin, calicheamicins, Sapaonaria officinalis inhibitor, as well as glucocorticoid and other chemotherapy agents, as well as radioisotopes such as At²¹¹, I¹³¹, I¹²⁵, Y⁹⁰, Re¹⁸⁶, Re¹⁸⁸, Sm¹⁵³, Bi²¹² or Bi²¹³, P³² and Lu (including Lu¹⁷⁷). Antibodies can also be conjugated to anticancer prodrug activating enzymes that can convert a prodrug into the active form.

One preferred drug useful in the present invention is maytansine or maytansinoids. Maytansine inhibits cell proliferation by inhibiting the formation of microtubules from tubulins. Maytansinoids are derivatives of maytansine. Both maytansine and maytansinoids are highly cytotoxic, but they are greatly limited for clinical use in cancer therapy due to poor selectivity for tumors. However, a high cytotoxicity enables them to be attractive drug moieties in ADCs. The structure shown below is deacetyl-maytansine.

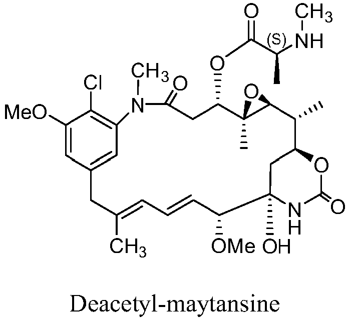

Another preferred drug useful in the present invention is auristatins. Auristatins are synthetic analogues of Dolastatin 10, which is a polypeptide isolated from marine mollusk Aplysia having biological activity. Dolastatin 10 inhibits tubulin polymerization by binding to tubulin at the same domain as anticancer drug vincristine. Dolastitin 10, auristatin PE, and auristatin E are all linear peptides having four amino acids, three of which are unique to the dolastatin class compounds, and a C-terminal amide group. Two representative auristatins, monomethyl auristatin E (MMAE) and monomethyl auristatin F (MMAF), are preferred drug moiety candidates for ADCs.

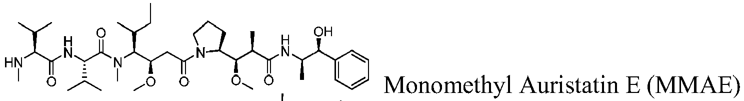

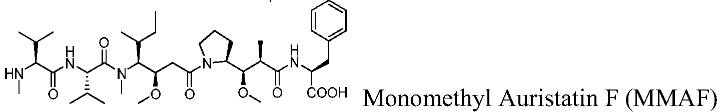

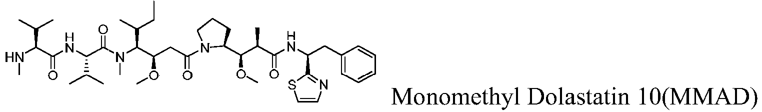

Another preferred drug of the present invention is microtubuleysin. Microtubulin was first isolated by the research team from slime cell cultures and is a highly effective cell growth inhibitor that works by inhibiting microtubule protein polymerization and inducing cell apoptosis. Microtubulin D in microtubule lysins is the most effective, with activity 10 to 100 times higher than most other microtubule protein regulators, including erythromycin, vinblastine, and paclitaxel. Paclitaxel and vinblastine are currently used for the treatment of various cancers, while epomycin derivatives are undergoing activity evaluation in clinical trials. The synthetic derivatives of microtubule lysin D will provide necessary information related to inhibition and critical binding interactions, and can exhibit superior properties as anticancer agents, either as isolated entities or as chemical warheads on targeted antibodies or ligands. Microtubulin D is a complex tetrapeptide that can be divided into four regions: Mep (D-N-methylpiperidinecarboxylic acid), Ile (isoleucine), Tuv (tubulvaline), and Tup (tubulphenylalanine), as shown in the following formula:

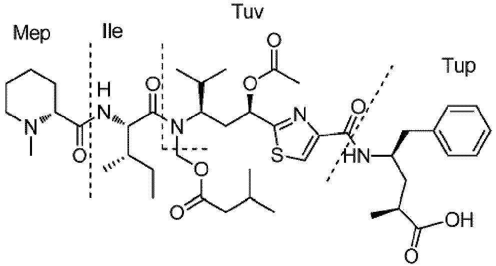

Another preferred drug of the present invention is a cryptophycin derivative derived from microorganisms that can inhibit microtubule polymerization. Cryptophycin is a novel anti-tumor active substance isolated from blue-green algae cultures that can inhibit microtubule formation and has activity against various tumors. Cryptophycin is a lipophilic compound containing 2 peptide bonds and 2 ester bonds, with 5 optically active centers and 1 epoxy group. The dipeptide diester bonds are all in a macrocyclic structure. The structures of Cryptophycin derivatives CP1 and CP2 are shown in the following formulas:

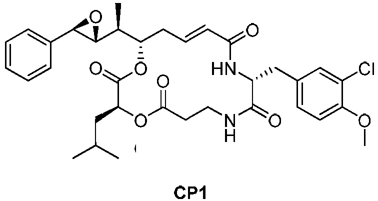

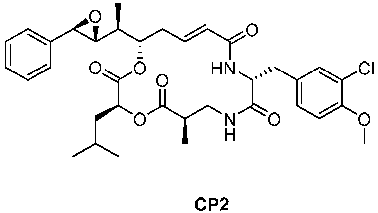

Another preferred drug of the present invention is the novel microtubule inhibitor Taltobulin (HTI-286, SPA-110). Taltobulin inhibits the polymerization of purified microtubules, interferes with intracellular microtubule organization, induces mitotic blockade, and induces cell apoptosis. Taltobulin is a potent inhibitor of cell proliferation, with an average IC50 of 2.5 nM against 18 human tumor cell lines. Compared with the currently used microtubule inhibitors, Taltobulin is not a suitable substrate for P-glycoprotein, and its structure is shown in the following figure:

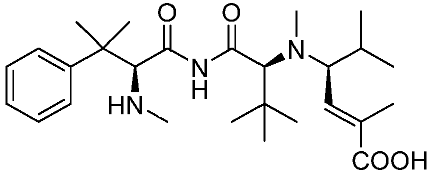

On the one hand, the drug is a derivative of camptothecin, SN-38. SN-38 is a bioactive metabolite of irinotecan hydrochloride (CPT-11) and a class of topoisomerase inhibitors. SN-38 causes the strongest inhibition of DNA topoisomerase I, dose- and time-dependent inhibition of DNA synthesis, and frequent DNA single strand breaks. The structure of SN-38 is shown in the following figure:

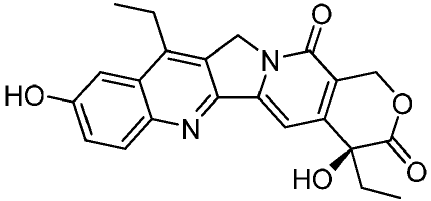

On the other hand, the drug is Exatecan, a derivative of camptothecin. It is a synthetic analogue of the topoisomerase I inhibitor camptothecin, with stronger activity than SN-38. It can cause the strongest inhibition of DNA topoisomerase I, dose- and time-dependent inhibition of DNA synthesis, and frequent DNA single strand breaks. The Exatecan structure is shown in the following formula:

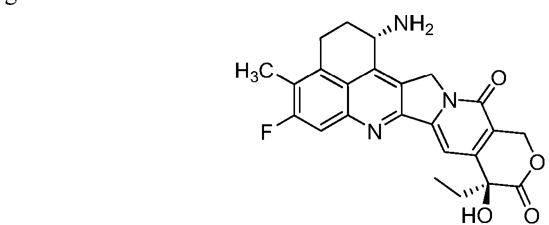

Another preferred drug of the present invention is alpha amanitin, which has the structure shown in the following figure. Alpha Amanitin is a fungal toxin derived from Amanita phalloides, a poisonous mushroom. It has a bicyclic octapeptide and can inhibit the transcription of eukaryotic RNA polymerase II and RNA polymerase III.

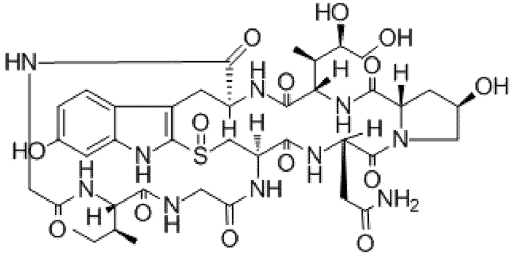

Another preferred drug of the present invention is a benzodiazepine antibiotic (duocarmycins, CC-1065, etc.) and other cyclopropyrrolidind-4-one (CPI) derivatives. This type of compound is an effective DNA small groove binding alkylating reagent. Cyclopropabenzindol-4-one (CBI) analogues have more stable chemical structures, higher biological activity, and are easier to synthesize compared to their parent compounds containing natural CPI alkylated subunits. A representative CBI derivative is the phenolic hydroxyl protected derivative CBI, which has weakened prodrug toxicity and enhanced water solubility (the CBI-*seco* class structural formula is shown in the following figure):

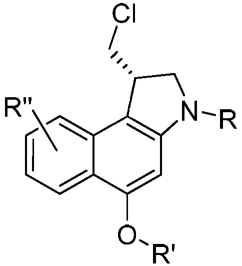

Another preferred drug of the present invention is pyrrolo [2,1-c] [1,4] benzodi azepine (PBDs) or PBD dimers. PBD is a natural product produced by Streptomyces, characterized by the ability to form non twisted covalent adducts in DNA grooves, specifically at the purine guanine purine sequence. The application of PBD as a partial small molecule strategy to target DNA sequences and as a novel anti-cancer and antibacterial drug has attracted increasing interest. The application of a flexible carbon chain to connect the C8/C8' hydroxyl groups of two PBD units results in a dimer with enhanced biological activity. PBD dimers are believed to generate sequence selective DNA damage, such as reverse 5'-Pu-GATC-Py-3' interchain cross-linking, leading to their biological activity. These compounds have been proven to be highly effective cytotoxic drugs and can serve as alternative drugs for antibody drug conjugates.

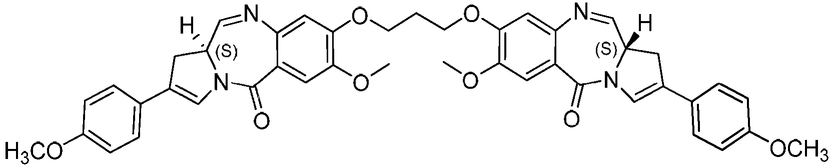

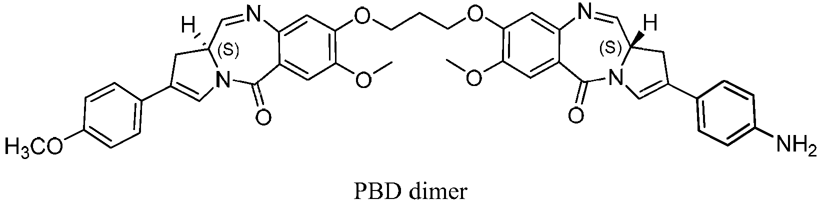

Another preferred drug of the present invention is a derivative of PNU-159682, which is the main active metabolite of Nemorubicin in human liver microsomes. Compared with MMDX and doxorubicin, PNU-159682 exhibits a 3000 folds increase in activity.

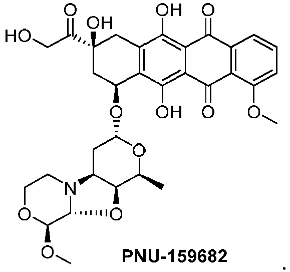

On the other hand, drugs are not limited to the categories mentioned above, but also include all drugs that can be used for antibody drug conjugates. And especially those that can coordinate through amide bonds with the linker, such as cell toxins that coordinate through basic amino groups (primary or secondary amines), such as the structure of cell toxins D1-D12 shown above.

### Linker

According to the mechanism of drug release within cells, "linker" or "linker of antibody drug conjugates" can be divided into two categories: unbreakable linker and breakable linker.

For antibody drug conjugates containing unbreakable linker, the drug release mechanism is as follows: after the conjugate binds to the antigen and is internalized by the cell, the antibody is enzymatically hydrolyzed in the lysosome, releasing an active molecule composed of small molecule drugs, linkers, and antibody amino acid residues. The resulting changes in the molecular structure of the drug do not weaken its cytotoxicity, but due to the charged nature of the active molecule (amino acid residues), it cannot penetrate neighboring cells. Therefore, such active drugs cannot kill adjacent tumor cells that do not express the targeted antigen (antigen negative cells) (bystander effect).

The breakable linker, as the name suggests, can break and release active drugs (small molecule drugs themselves) within the target cell. Breakable linkers can be divided into two main categories: chemically unstable linkers and enzymatically unstable linkers. Chemical unstable linkers can selectively break due to differences in plasma and cytoplasmic properties. Such properties include pH value, glutathione concentration, etc. pH sensitive linkers are commonly referred to as acid break linkers. Such linkers are relatively stable in the neutral environment of blood (pH 7.3-7.5), but will be hydrolyzed in weakly acidic endosomes (pH 5.0-6.5) and lysosomes (pH 4.5-5.0). The first generation of antibody drug conjugates mostly used such linkers, such as hydrazones, carbonates, acetals, and ketones. Due to the limited plasma stability of acid cleaved linkers, antibody drug conjugates based on such linkers typically have a short half-life (2-3 days). This shorter half-life to some extent limits the application of pH sensitive linkers in the new generation of antibody drug conjugates.

For glutathione sensitive linker, also known as disulfide bond linkers. Drug release is based on the difference between the high concentration of intracellular glutathione (in the millimolar range) and the relatively low concentration of glutathione in the blood (in the micromolar range). This is especially true for tumor cells, whose low oxygen content leads to increased activity of reductases, resulting in higher concentrations of glutathione. Disulfide bonds have thermodynamic stability and therefore exhibit good stability in plasma.

Enzyme unstable linker, such as peptide linkers, can better control drug release. Peptide linkers can be effectively cleaved by proteases within the lysosome, such as cathepsin B or fibrinolytic enzymes (which have increased levels in some tumor tissues). This peptide linkage is considered to be very stable in plasma circulation, as inappropriate pH values outside the cell and serum protease inhibitors often render proteases inactive. Due to its high plasma stability and good intracellular cleavage selectivity and effectiveness, enzyme unstable linkers are widely used as breakable linkers for antibody drug conjugates. Typical enzyme unstable linkers include Val Kit (VC), Val Ala (VA), Gly Gly Phe Gly (GGFG), etc.

The self-releasing linker is generally embedded between the breakable linker and the active drug, or is itself a part of the breakable linker. The mechanism of action of the self-releasing linker is that when the breakable linker is broken under appropriate conditions, the self-releasing linker can spontaneously undergo structural rearrangement, thereby releasing the active drug connected to it. Common suicide linkers include para aminobenzyl alcohol (PAB) and β - glucuronide.

### Application

The present invention further provides use of the antibody of the present invention, for example, for preparation of a diagnostic agent, or for preparation of a medicine for preventing and/or treating a HER2-related disease. The HER2-related disease includes tumorigenesis, tumor growth and/or metastasis, a tumor resistance-related disease, inflammation, a metabolism-related disease, etc.

Use of the nanobody, fusion protein, NDC or CAR-T according to the present invention includes but is not limited to:
(i) diagnosis, prevention and/or treatment of tumorigenesis, tumor growth and/or metastasis, particularly, for a tumor with HER2 high expression; wherein the tumor includes (but is not limited to): breast cancer (e.g. triple negative breast cancer), lung cancer (such as non-small cell lung cancer), pancreatic cancer, malignant glioma, gastric cancer, liver cancer, esophageal cancer, kidney cancer, colorectal cancer, bladder cancer, prostate cancer, endometrial cancer, ovarian cancer, cervical cancer, leukemia, bone marrow cancer, angiosarcoma, etc.; preferably triple negative breast cancer, non-small cell lung cancer, pancreatic cancer, malignant glioma; and more preferably triple negative breast cancer and/or non-small cell lung cancer;
(ii) diagnosis, prevention and/or treatment of an autoimmune disease; wherein the autoimmune disease includes (but are not limited to): systemic lupus erythematosus, rheumatoid arthritis, ulcerative colitis, type I diabetes, psoriasis, multiple sclerosis;
(iii) diagnosis, prevention and/or treatment of inflammation; wherein the inflammation includes (but is not limited to): rheumatic arthritis, osteoarthritis, ankylosing spondylitis, gout, Lytle syndrome, psoriasis arthritis, infectious arthritis, tuberculous arthritis, viral arthritis, fungal arthritis, glomerular Nephritis, systemic lupus erythematosus, Crohn's disease, ulcerative colitis, acute lung injury, chronic obstructive pulmonary disease, and idiopathic pulmonary fibrosis;
(iv) diagnosis, prevention and/or treatment of a metabolism-related disease, wherein the metabolism-related disease includes (but is not limited to): diabetes, diet-induced obesity, adipose inflammation.

### Pharmaceutical composition

The invention further provides a composition. In the preferred examples, the composition is a pharmaceutical composition comprising the antibody, or an active fragment, a fusion protein or an NDC thereof, or a corresponding CAR-T cell, and a pharmaceutically acceptable carrier. In general, these substances may be formulated in a non-toxic, inert and pharmaceutically acceptable aqueous carrier medium, wherein the pH is generally about 5-8, preferably, pH is about 6-8, although the pH value may be varied depending on the nature of the substances to be formulated and the condition to be treated. The formulated pharmaceutical composition may be administered by conventional routes, including (but not limited to): intratumoral, intraperitoneal, intravenous, or topical administration.

The antibody of the present invention can also be used for cell therapy by expressing the nucleotide sequence in a cell, for example, the antibody is used for chimeric antigen receptor T cell immunotherapy (CAR-T) and the like.

The pharmaceutical composition of the present invention can be directly used for binding to HER2 protein molecule, and thus can be used for preventing and treating diseases such as tumors. In addition, other therapeutic agents can also be used simultaneously.

The pharmaceutical composition according to the present invention comprises a safe and effective amount (e.g. 0.001-99 wt%, preferably 0.01-90 wt%, preferably 0.1-80 wt%) of the monoclonal antibody according to the present invention (or a conjugate thereof) and a pharmaceutically acceptable carrier or excipient. Such carriers include, but are not limited to, saline, buffer solution, glucose, water, glycerin, ethanol or the combination thereof. The pharmaceutical preparation should be matched to the method of administration. The pharmaceutical composition of the present invention can be prepared in the form of injection, for example, prepared by a conventional method using physiological saline or an aqueous solution containing glucose and other adjuvants. Pharmaceutical compositions such as injections and solutions are preferably prepared under sterile conditions. The dosage of active ingredient is therapeutically effective amount, for example from about 1 microgram per kilogram body weight to about 5 milligrams per kilogram body weight per day. Further, the polypeptide of the present invention can also be used in combination with the other therapeutic agents.

When a pharmaceutical composition is used, a safe and effective amount of the immunoconjugate is administered to a mammal, wherein the safe and effective amount is usually at least about 10 micrograms per kilogram of body weight, and in most cases does not exceed about 50 mg/kg body weight, preferably the dose is about 10 micrograms/kg body weight to about 20 mg/kg body weight. Of course, the particular dose should also depend on various factors, such as the route of administration, patient healthy status, which are well within the skills of an experienced physician.

As for the NDC, the nanobody-drug conjugate provided by the present invention can target a specific cell population and bind to a specific protein (antigen) on cell surface, thereby releasing the drug into the cell in an active form through endocytosis or drug infiltration of the conjugate. Thus, the nanobody-drug conjugate of the invention can be used to treat diseases of interest, and the antibody-drug conjugate mentioned above can be administered to a subject (e.g., a human) by a suitable route in a therapeutically effective amount. A subject in need of treatment can be a patient at risk of having or suspected of having a condition associated with the activity or amount of expression of a particular antigen. Such patients can be identified by routine physical examination.

When the nanobody-drug conjugate as described herein is used as the therapeutic agent, it can be delivered by methods conventional in the art. For example, it can be introduced into cells using liposomes, hydrogels, cyclodextrins, biodegradable nanocapsules, or bioadhesive microspheres. Alternatively, nucleic acids or vectors can be delivered *in situ* by direct injection or by use of an infusion pump.

### The main advantages of the present invention include:

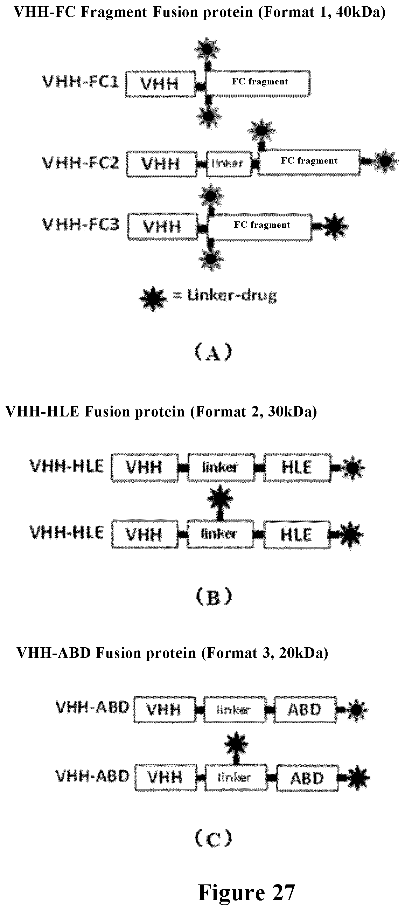

1. The nanobody of the present invention has excellent biological activity and specificity, six preferred antibodies 1-C10, 2-C07, 1-G07, 1-B05, 1-C09, 2-F03 have a high affinity, and the EC₅₀ determined by ELISA is 0.004-0.017µg/mL (0.05-0.128nM).
2. The binding constants determined by surface plasmon resonance (SPR) ranged from 4.613nM-23.24nM.
3. It has good binding affinity for HER2 in tumor cells, and FACS analysis shows that its minimum effective binding concentration is <0.1µg/mL, with an EC₅₀ of 0.1-0.2µg/mL. It can be used as a therapeutic antibody targeting HER2.
4. The nanobody fusion protein of the present invention is suitable for preparing nanobody drug conjugates (NDCs).
5. The preferred NDC of the invention has excellent anti-tumor activity in vitro, and the IC₅₀ values of proliferation inhibition for gastric cancer N87 cells, breast cancer SK-BR-3 cells, and breast cancer BT474 cells are all lower than 0.03nM.
6. The preferred NDC of the present invention has excellent in vivo anti-tumor activity, and can significantly inhibit tumor growth and lead to tumor regression after a single intravenous injection of 3mg/kg and 1mg/kg.
7. According to the design of Figure 27 of the present invention, TF nanobody drug conjugates with desired DAR values can be conveniently prepared, and their crosslinking reaction formulas are shown in Figure 28 1a, 1b, 1c or 1d, respectively.

### Example 1 Discovery and preparation of nanobodies targeting human HER2

The antigen was prepared by referencing the NCBI: NP_004439.2 amino acid sequence (Met 1-Thr 652) and obtaining the carbon terminal polyhistidine tagged human HER2 His6 protein (Sinobiological, Catlog # 10004-H08H) through HEK293 cell expression system.

The HER2 protein prepared above was used to immunize Alpaca, with an interval of 21 days between immunizations. After the last immunization, partial peripheral blood cells (PBMCs) were collected 10 days later; a yeast display library was constructed through PBMC isolation and VHH antibody fragment cloning. By inducing expression, FACS sorting, and repeated screening of the display library, six highly active nanobodies with independent CDR sequences were obtained from the original display library, including nine independent VHH sequences: 1-C10, 2-C07, 1-B05, 1-C09, 2-F03, and 1-G07. The above variable region is fused and expressed with the human constant region (hIgG1 Fc segment) to prepare purified chimeric nanobodies.

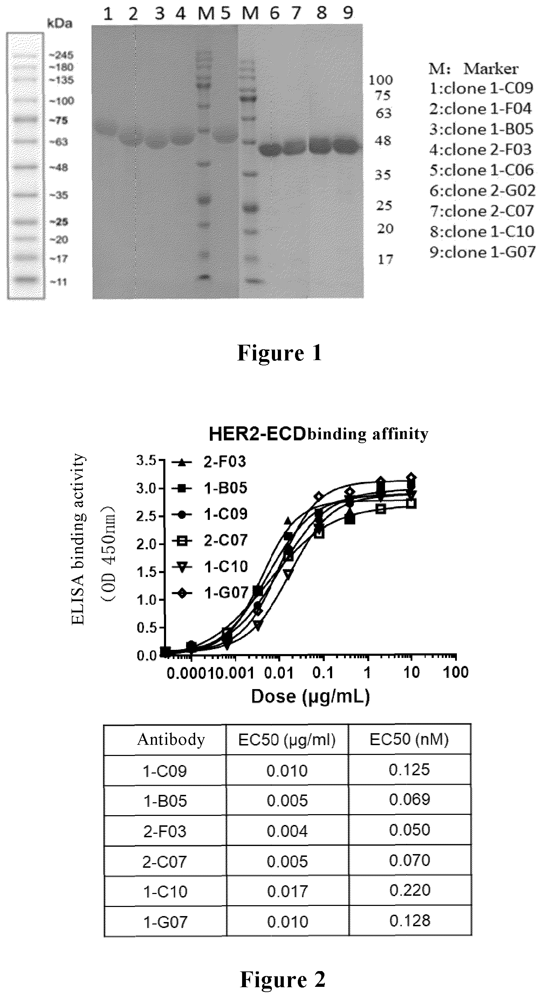

As shown in **Figure 1****,** SDS-PAGE electrophoresis was used to detect multiple chimeric antibodies prepared based on the title, all of which showed the expected protein purity and molecular weight.

### Example 2 Determination of affinity of nanobodies for human HER2 antigen by ELISA

HER2 protein was diluted to 1µg/mL with coating solution, coated onto ELISA plate, 100µL/well, 4°C, overnight. The excess antigens were washed off. The plate was blocked with 1% BSA at room temperature for 2 hours, then 5-fold gradient diluted monoclonal antibodies were added at 100µL/well, and the plate was incubated at room temperature for 1 hour; the unbound antibodies were washed off, and suitable concentration of horseradish peroxidase labeled Staphylococcus protein A was added at 100µL/well, and the plate was incubated at room temperature for 0.5 hours. The unbound secondary antibody was washed off. TMB colorimetric solution was added and reacted for about 15 minutes. 1M HCl at 50µL/well was added to terminate the colorimetric reaction, then the absorbance was measured at 450nm and the data was analyzed.

The detection results are shown in **Figure 2**1-C10, 2-C07, 1-B05, 1-C09, 2-F03, and 1-G07 had strong affinity for human HER2, with EC₅₀ values of 0.017µg/mL, 0.005µg/mL, 0.005µg/mL, 0.01µg/mL, 0.004µg/mL, and 0.01µg/mL, respectively.

### Example 3 Determination of SPR affinity of nanobodies for human HER2 antigen

Surface plasmon resonance (SPR) detection was performed and Protein A sensor was used to solidify the antibody at a curing height of approximately 1.5nM. The buffer was PBST (PBS+0.02% Tween20), and HER2 protein samples were diluted to 500, 250, 125, 62.5, 31.3, and 0 nM. Affinity detection: equilibrium for 60 seconds, binding for 180 seconds, dissociation for 180 seconds, detection at 25 °C, and kinetic characterization analysis was performed using the ForteBio OCTET R2 system.

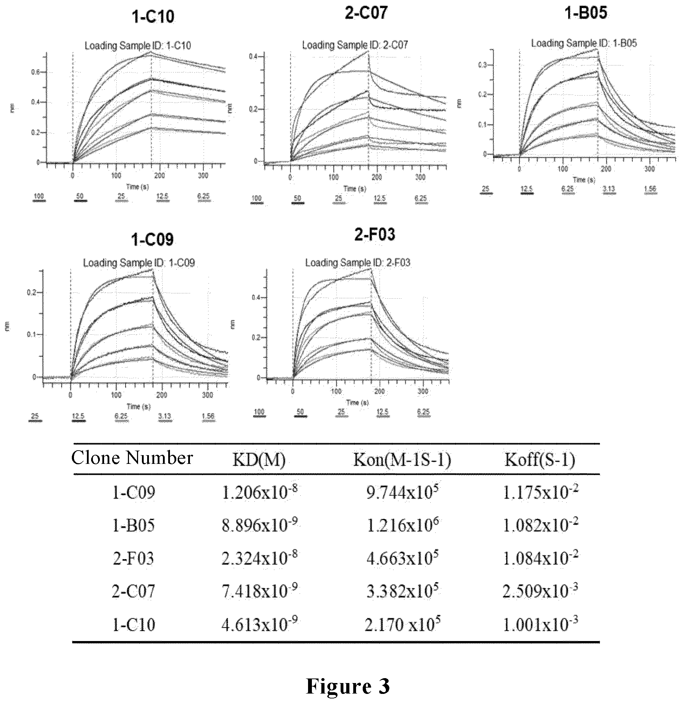

The detection results are shown in **Figure 3****.** 1-C10, 2-C07, 1-B05, 1-C09, and 2-F03 had strong binding affinity for HER2, with binding constants (KD) of 4.613nM, 7.418nM, 8.896nM, 12.06nM, and 23.24nM, respectively.

### Example 4 Determination of binding affinity of nanobody to HER2 on tumor cell surface

HER2 overexpressing gastric cancer cell line NCI-N87 was used as the target cell. 100µL of the test antibody diluted in a 10 folds gradient was used as the primary antibody. It was mixed with 1×10⁵ tumor cells suspended in 100µL RPMI-1640 serum-free medium at final concentrations of 10µg/mL, 1µg/mL, and 0.1µg/mL. The cells were then incubated at 4°C for 1 hour, washed twice with PBS to remove unbound primary antibodies, and incubated with 200µL, 2µg/mL, PE labeled secondary antibodies at 4°C for 30 minutes. The cells were washed twice with PBS to remove unbound secondary antibodies. Finally, the cells were resuspended in 200µL PBS and analyzed by flow cytometry to determine the binding affinity of the test antibody to HER2 on the corresponding cell surface.

The cell binding affinity was obtained by FACS detection with further concentration gradient dilution.

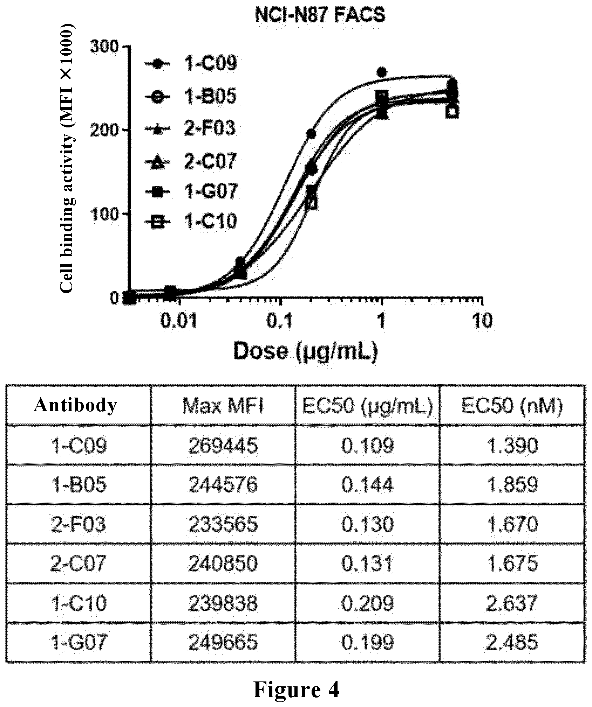

The detection results are shown in **Figure 4****.** The binding affinities EC₅₀ of 1-C10, 2-C07, 1-B05, 1-C09, 2-F03, and 1-G07 to NCI-N87 cells are 0.209 µg/mL, 0.131 µg/mL, 0.144 µg/mL, 0.109 µg/mL, 0.130 µg/mL, and 0.199 µg/mL, respectively.

The above results indicate that the HER2 nanobody in this example has good binding activity with human HER2 antigen.

### Example 5 Antitumor activity of HER2 nanobody in a nude mouse transplanted tumor model

N87 cells were resuspended in sterile PBS and the concentration was adjusted to 100×10⁶/ml. An equal volume of cell suspension was taken and mixed with 1mg/ml antibody to incubate on ice for 1 hour. 100µL of antibody/cell suspension (containing 5×10⁶ cells, 50µg of antibody) was taken, and 100µL of standard concentration matrix gel was added to inoculate subcutaneously on the side back of female immunodeficient mice (Balb/c nude, 5 weeks old) (n=6). Tumor volume and mouse body weight were measured 2-3 times a week based on tumor growth status and recorded to draw a tumor growth curve. After the experiment, the experimental data was collected and analyzed, and tumor growth curves and nude mouse weight change curves were plotted. Subcutaneous transplanted tumors were surgically removed and weighed, with the tumor volume (V) calculated as V=L×W2/2, where L and W represent the length and width of the tumor, respectively.

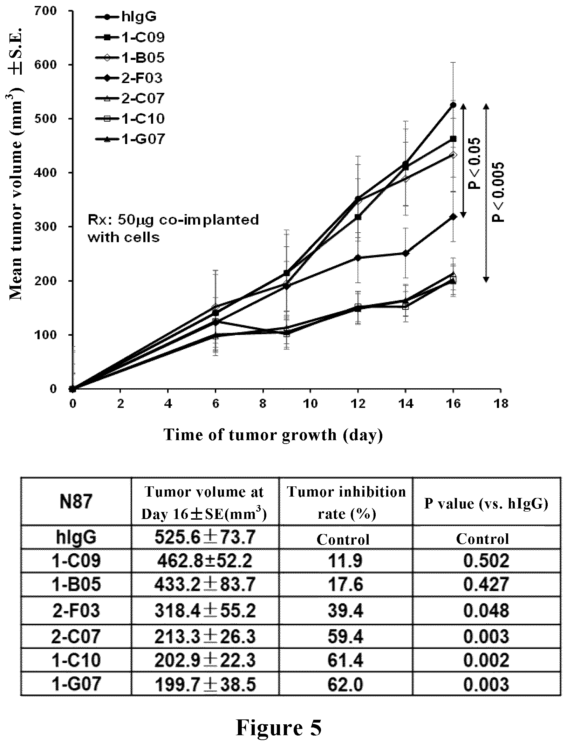

As shown in **Figure 5****,** the HER2 nanobodies 2-F03, 2-C07, 1-C10, and 1-G07 of the present invention inhibited tumor growth in vivo, with 2-C07, 1-C10, and 1-G07 exhibiting significant tumor growth inhibitory effects.

### Example 6 Immunohistochemical experiment of tumor tissue

The N87 subcutaneous xenograft tumor was fixed in 4% formalin, embedded in paraffin, and sliced. Immunohistochemical staining (IHC) and TSA staining were performed according to the standard experimental protocol. The IHC staining result images were obtained using a scanner (Olympus, #VS200, Tokyo, Japan). The TSA staining result images were obtained using a Leica microscope (DMI4000D). The obtained images were quantified using ImageJ software and normalized to the control group, followed by statistical analysis using GraphPad Prism8 software. The antibody used is anti-p-AKT antibody (Abcam, Cat # ab81283).

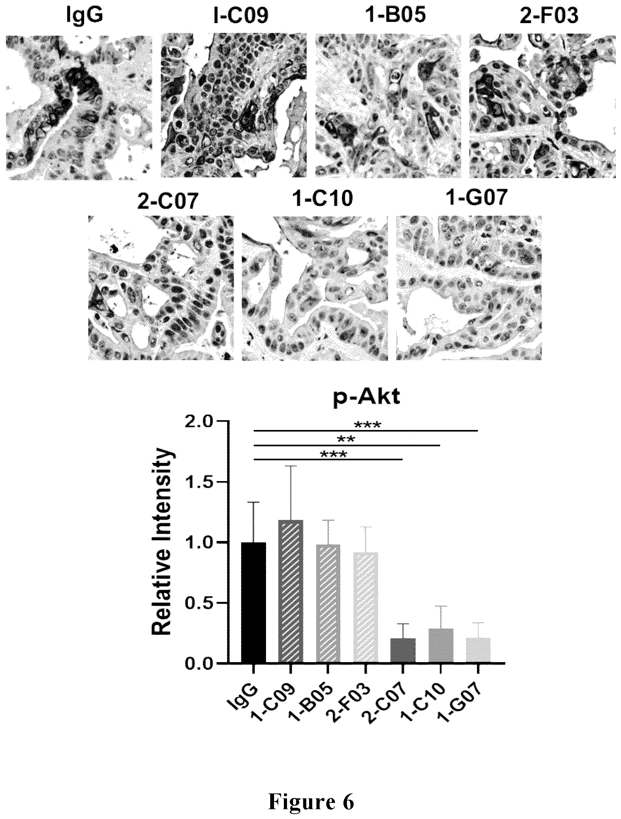

As shown in **Figure 6****,** the HER2 nanobodies 2-C07, 1-C10, and 1-G07 of the present invention significantly inhibited p-AKT and suppressed AKT signaling pathway transduction in vivo.

### Example 7 Batch expression production and purification of HER2 nanobody and fusion proteins

The designed 1-G07-FCWT and 1-G07-ABD sequences were cloned into recombinant fusion protein expression vectors, and suspend them in serum-free medium for 6 days after transient transfer to HEK293. Collect the culture supernatant for purification, protein quantification, and SDS-PAGE detection.

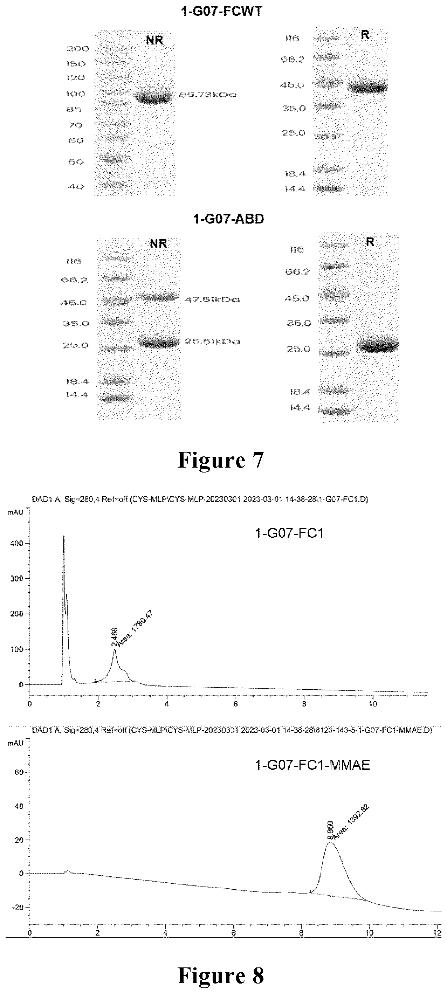

As shown in **Figure 7****,** the antibody fusion proteins derived from 1-G07-FCWT and 1-G07-ABD can be efficiently expressed, produced, and purified.

### Example 8 Preparation of HER2 nanobody-ve-MMAE conjugate

The antibody protein stock solution was diluted with 50mM potassium dihydrogen phosphate sodium hydroxide (KH₂PO₄-NaOH)/150mM sodium chloride (NaCl)/1mM diethylenetriaminepentaacetic acid (DTPA), pH 7.4 reaction buffer to 5mg/mL. 3-4 times the excess molar ratio of tris (2-carboxyethyl) phosphine hydrochloride (TCEP) was added, and the reaction solution was stirred at 25°C for 2.5 hours.

The above reaction solution was cooled to 0-10°C. An appropriate amount of diethylacetamide (DMA) was added without purification, and then 6 times the excess molar ratio of mc-vc-MMAE (10mg/ml pre dissolved in DMA) was added to ensure that the volume proportion of DMA in the reaction system does not exceed 10%. The reaction system was stirred at 10-25°C for 2 hours for coupling.

The coupling reaction mixture was filtered and purified with the histidine acetic acid/sucrose gel of pH 6.0 using a desalting column, and the peak samples were collected according to the UV280 ultraviolet absorption value, then sterilized through a 0.22micron pore size filtration device and stored at -80°C.

For naked antibodies and MMAE conjugates, methods such as hydrophobic interaction chromatography (HIC) and molecular exclusion chromatography (SEC) are used to detect purity and coupling degree. For antibody dimer conjugates, the target drug loading DAR is 4; for antibody ADB monomer conjugates, the target drug loading DAR is 1.

As shown in **Figure 8****,** the 1-G07-FC1-MMAE conjugate showed a single peak upon HIC detection analysis, which is expected to be the DAR4 product of dimer site coupling.

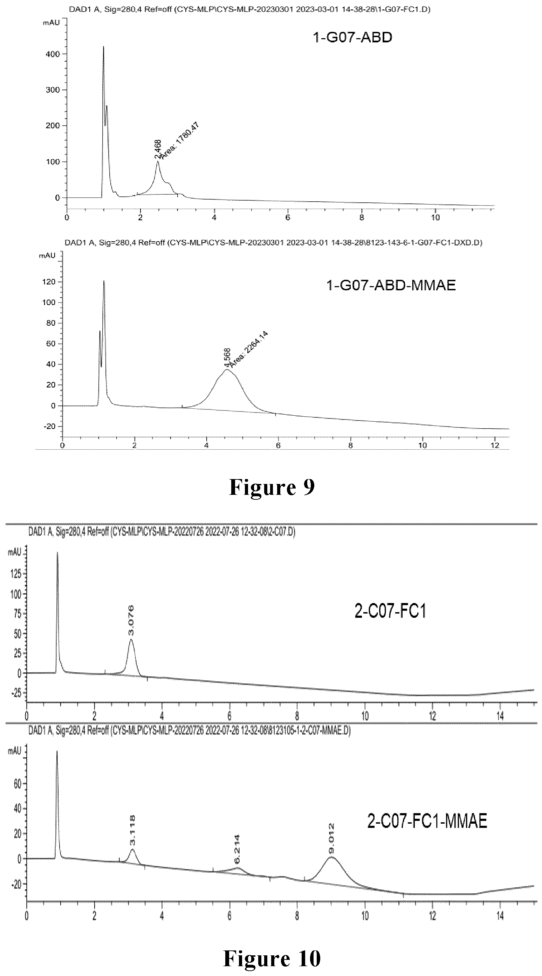

As shown in **Figure 9****,** the 1-G07-ABD-MMAE conjugate showed a single peak upon HIC detection analysis, which is expected to be the DAR1 product of monomer site coupling.

As shown in **Figure 10****,** the 2-C07-FC1-MMAE conjugate showed a main peak through HIC detection analysis, which is expected to be the DAR4 product of dimer site coupling.

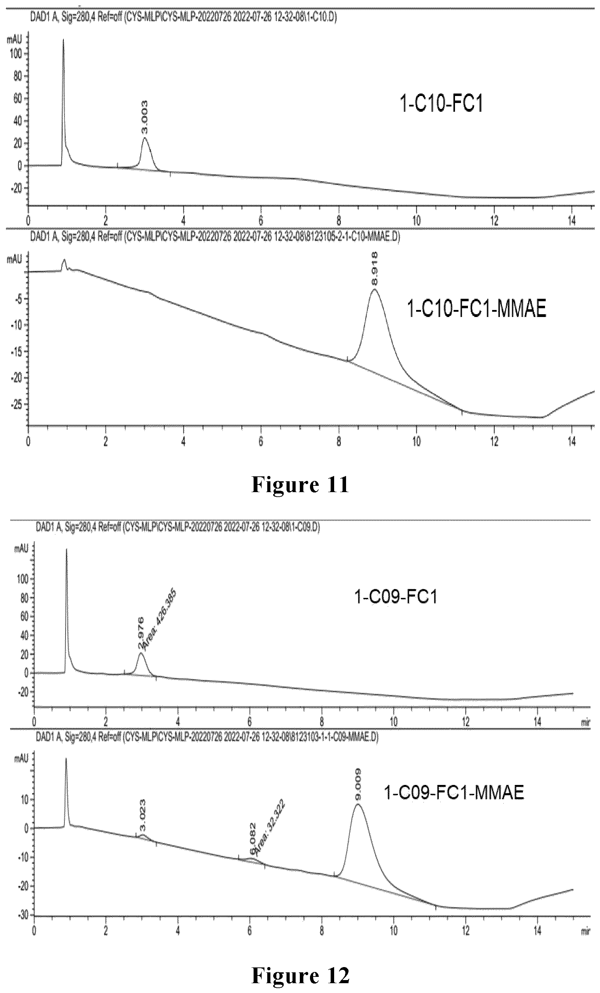

As shown in **Figure 11,** the 1-C10-FC1-MMAE conjugate exhibited a single peak upon HIC detection analysis, which is expected to be the DAR4 product of dimer site coupling.

As shown in **Figure 12****,** the 1-C9-FC1-MMAE conjugate exhibited a single peak upon HIC detection analysis, which is expected to be the DAR4 product of dimer site coupling.

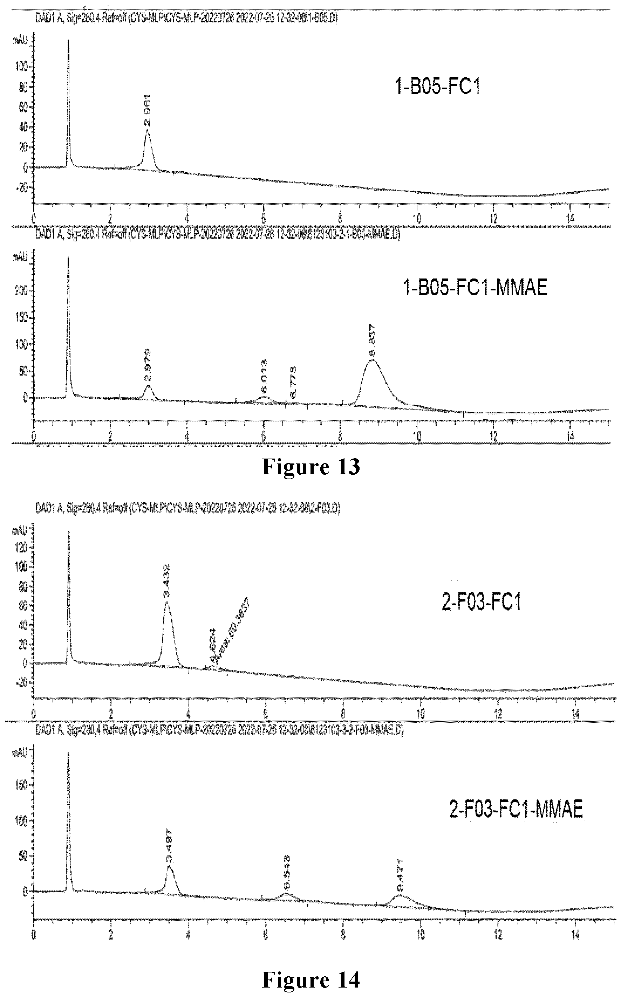

As shown in **Figure 13****,** the 1-B05-FC1-MMAE conjugate showed a main peak through HIC detection analysis, which is expected to be the DAR4 product of dimer site coupling.

As shown in **Figure 14****,** the 2-F03-FC1-MMAE conjugate showed three peaks (including naked antibody peak) after HIC detection analysis, indicating incomplete coupling and only partial DAR4 products.

### Example 9 Preparation of HER2 nanobody-GGFG-DXd conjugate

The 1-G07-FC1 naked antigen solution was replaced with a reaction buffer of 50mM sodium dihydrogen phosphate disodium hydrogen phosphate (NaH₂PO₄-Na₂HPO₄)/150mM sodium chloride (NaCl)/2mM ethylenediaminetetraacetic acid (EDTA), pH 7.0, to a concentration of 10mg/mL. 2.2 times the excess molar ratio of tris (2-carboxyethyl) phosphine hydrochloride (TCEP) was added, and the reaction mixture was stirred at 25°C for 4-6 hours. The above reaction solution was cooled to room temperature. An appropriate amount of diethylacetamide (DMA) was added, and then a 4-fold excess molar ratio of compound-GGFG-DXd (purchased from Shanghai Haoyuan Chemical, 10mg/ml pre dissolved in DMA) was added, ensuring that the volume proportion of DMA in the reaction system does not exceed 10%. The reaction system was stirred at 25°C for 1-2 hours for coupling. Excess small molecules were removed by using G25 desalination column or directly ultrafiltrated with filter tube, replaced to PBS/8% sucrose, pH 7.4 buffer, and stored at -80°C. Then, the antibody conjugate was sterilized using a filtration device with a pore size of 0.22 microns and stored at -80°C. The resulting antibody conjugate was named 1-G07-DXd.

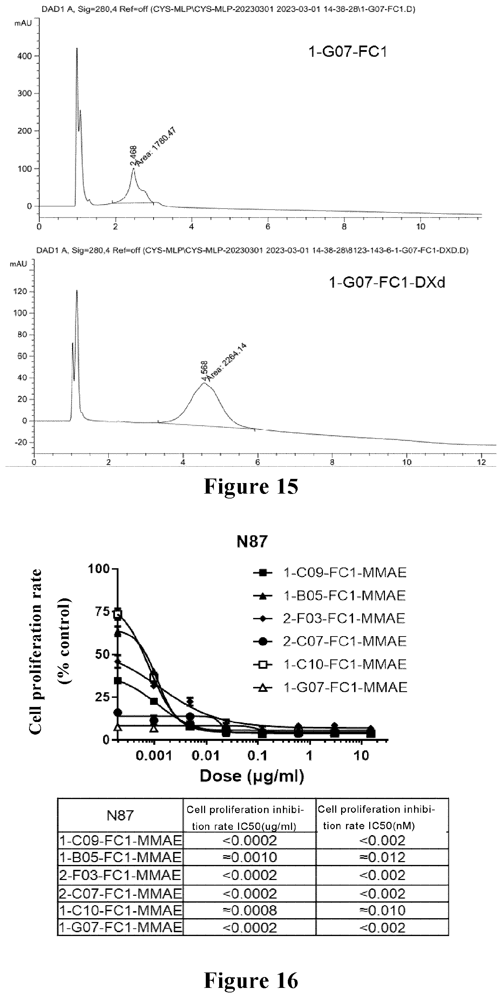

As shown in **Figure 15****,** the 1-G07-FC1-Dxd conjugate showed a single peak upon HIC detection analysis, which is expected to be the DAR4 product of dimer site coupling.

### Example 10 In vitro anti-tumor activity of HER2 nanobody conjugate (HER2-NDC)

The cell lines used in this example were purchased from the American Type Culture Collection (ATCC) or Nanjing Kebai Biotechnology Co., Ltd. and cultured according to the corresponding instructions, including NCI-N87, SK-BR-3, BT-474, HCC1954.

Cell proliferation test: the cells in logarithmic growth phase were inoculated into 96 well cell culture plates according to the appropriate number of cells based on their growth rate, with 150µL of culture medium per well, and incubated at 37°C and 5% CO₂ for 5 hours. Then, HER2 NDCs of different concentrations were added and corresponding wells as well as solvent control and blank control wells were set up. After 6-9 days of treatment, the culture medium was removed and MTS reaction solution was added (MTS powder purchased from Promega, G1111); PMS powder was purchased from Sigma, P9625, at a concentration of 100µL/well. After reacting at 37 °C to the appropriate color depth, the cell viability (OD490nm) of each group was measured using a multifunctional enzyme-linked immunosorbent assay (BioTek Synergy II), and the cell survival rate was calculated according to the following formula: Survival rate= (OD_{administration}-OD_{blank})/(OD_{control}-OD_{blank}) × 100%. The above data was analyzed using GraphPad Prism8 software and the IC₅₀ values of different drugs on different cell lines were calculated.

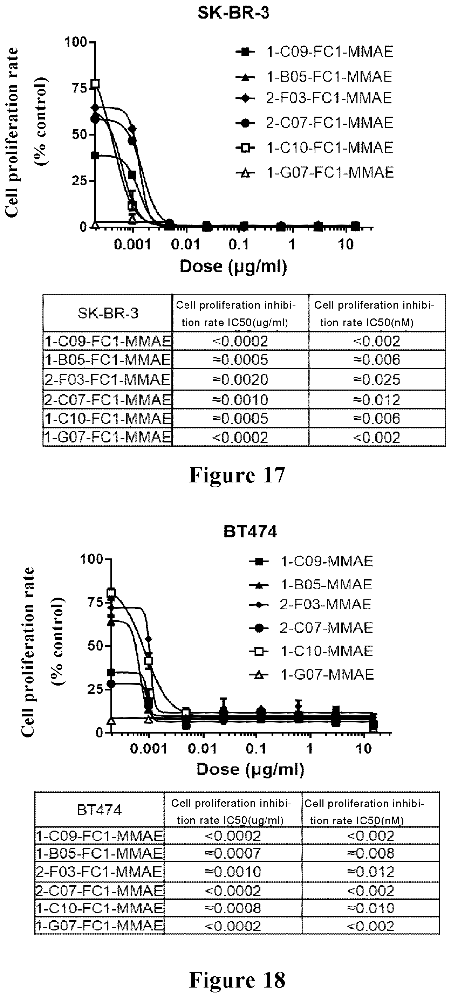

The results shown in **Figure 16****,** **Figure 17** and **Figure 18** show that the six HER2 nanobody-drug conjugates of the invention, 1-C09-FC1-MMAE, 1-B05-FC1-MMAE, 2-F03-FC1-MMAE, 2-C07-FC1-MMAE, 1-C10-MMAE, 1-G07-FC1-MMAE, 1-G07-FC1-MMAE, showed strong cell proliferation inhibitory activity against HER2 positive gastric cancer NCI-N87, breast cancer SK-BR-3, and breast cancer BT-474 cells, with IC₅₀ values lower than 0.03nM.

**Table-3** summarizes the IC₅₀ values of some cell proliferation inhibition tests.

**Table-3: In vitro anti-tumor activity of HER2 nanobody-drug conjugate**

| HER2-NDC | **SK-BR-3** | | **BT474** | | **NCI-N87** | |
|---|---|---|---|---|---|---|
| | IC50(µg/ml) | IC50(nM) | IC50(µg/ml) | IC50(nM) | IC50(µg/ml) | IC50(nM) |
| 1-C09-FC1-MMAE | <0.0002 | <0.0002 | <0.0002 | <0.0002 | <0.0002 | <0.0002 |
| 1-B05-FC1-MMAE | ≈0.0005 | ≈0.006 | ≈0.0007 | ≈0.008 | ≈0.001 | ≈0.012 |
| 2-F03-FC1-MMAE | ≈0.002 | ≈0.025 | ≈0.001 | ≈0.012 | <0.0002 | <0.0002 |
| 2-C07-FC1-MMAE | ≈0.001 | ≈0.012 | <0.0002 | <0.0002 | <0.0002 | <0.0002 |
| 1-C10-FC1-MMAE | ≈0.0005 | ≈0.006 | ≈0.0008 | ≈0.010 | ≈0.0008 | ≈0.010 |
| 1-G07-FC1-MMAE | <0.0002 | <0.0002 | <0.0002 | <0.0002 | <0.0002 | <0.0002 |

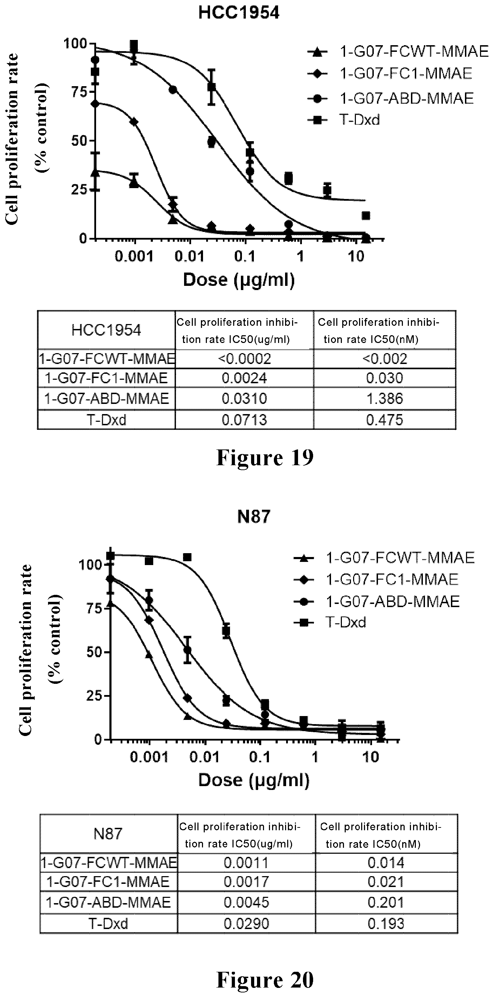

The results shown in **Figures 19 and 20** show that the HER2 nanobody-drug conjugate of the invention has a strong inhibitory activity on cell proliferation of HER2 positive gastric cancer NCI-N87 and breast cancer HCC1954 cells in 1-G07-FC1-MMAE, 1-G07-FCWT-MMAE, 1-G07-ABD-MMAE matching different domains, which is stronger than the control antibody-drug conjugate T-Dxd.

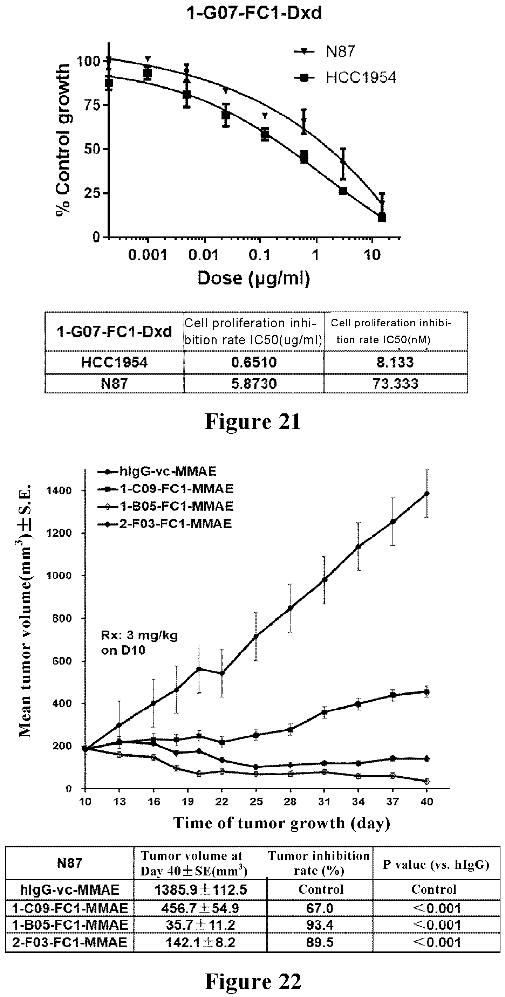

As shown in **Figure 21****,** the preferred HER2 nanobody-drug conjugate 1-G07-FC1-Dxd of the invention can inhibit the proliferation activity of HER2 positive gastric cancer NCI-N87 and breast cancer HCC1954 cells.

**Table-4** summarizes the IC₅₀ values of some cell proliferation inhibition tests.

**Table-4: In vitro anti-tumor activity of HER2 nanobody-drug conjugate**

| HER2-NDC | **HCC1954** | | **N87** | |
|---|---|---|---|---|
| | IC50(µg/ml) | IC50(nM) | IC50(µg/ml) | IC50(nM) |
| 1-G07-FCWT-MMAE | <0.0008 | <0.01 | 0.0011 | 0.014 |
| 1-G07-FC1-MMAE | 0.0024 | 0.03 | 0.0017 | 0.021 |
| 1-G07-ABD-MMAE | 0.031 | 1.39 | 0.0045 | 0.201 |
| T-Dxd | 0.0713 | 0.475 | 0.029 | 0.193 |

### Example 10 In vivo anti-tumor activity of HER2 nanobody conjugate (HER2-NDC)

200µL of suspension of PBS containing 5×10⁶ N87 cells and matrix gel (PBS: standard concentration matrix gel=1:1) was inoculated subcutaneously on the back of female immunodeficient mice (Balb/c nude, 5-6 weeks old). When the tumor volume reaches 200-400 mm³ and significant tumor growth can be observed, the nude mice were randomly divided into groups based on tumor volume size and body weight (n=6-8), and doses of 3mg/kg, 1mg/kg, and 0.3mg/kg were administered via tail vein once, or once a week for two weeks, with hIgG or hIgG1-vc-MMAE as negative controls. The tumor volume and nude mouse weight were measured 2-3 times a week and recorded to draw the tumor growth curve. After the experiment, the experimental data was collected and analyzed, and tumor growth curves and nude mouse weight change curves were plotted. Subcutaneous transplanted tumors were surgically removed and weighed, with the tumor volume (V) calculated as V=L×W2/2, where L and W represent the length and width of the tumor, respectively.

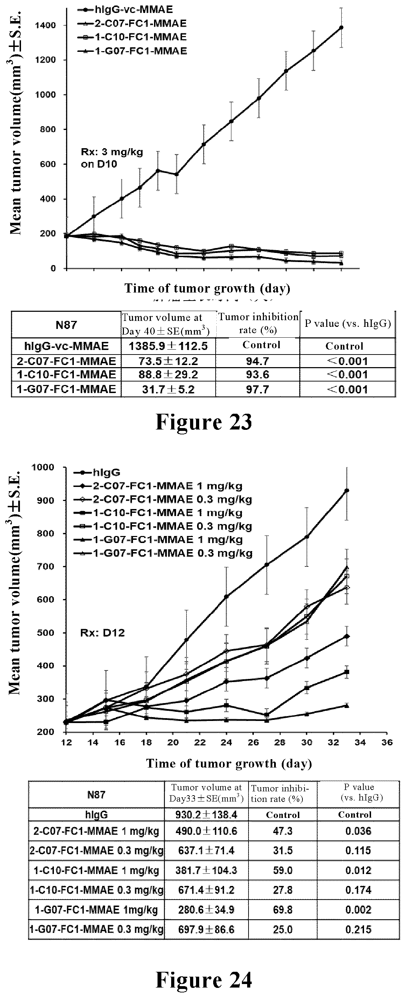

As shown in **Figures 22** **and** **23****,** the preferred HER2 nanobody drug conjugates 1-C09-FC1-MMAE, 1-B05-FC1-MMAE, 2-F03-FC1-MMAE, 2-C07-FC1-MMAE, 1-C10-MMAE, and 1-G07-FC1-MMAE of the present invention showed significant anti-tumor activity against the N87 tumor model after a single intravenous injection of 3mg/kg, wherein tumors remained in a growth inhibited state after 30 days of administration of 1-B05-FC1-MMAE, 2-C07-FC1-MMAE, 1-C10-MMAE, and 1-G07-FC1-MMAE.

As shown in **Figure 24****,** in repeated experiments, single administration of 2-C07-FC1-MMAE, 1-C10-MMAE, and 1-G07-FC1-MMAE still showed significant anti-tumor activity at a dose of 1mg/kg, and also had a certain anti-tumor effect at a dose of 0.3mg/kg.

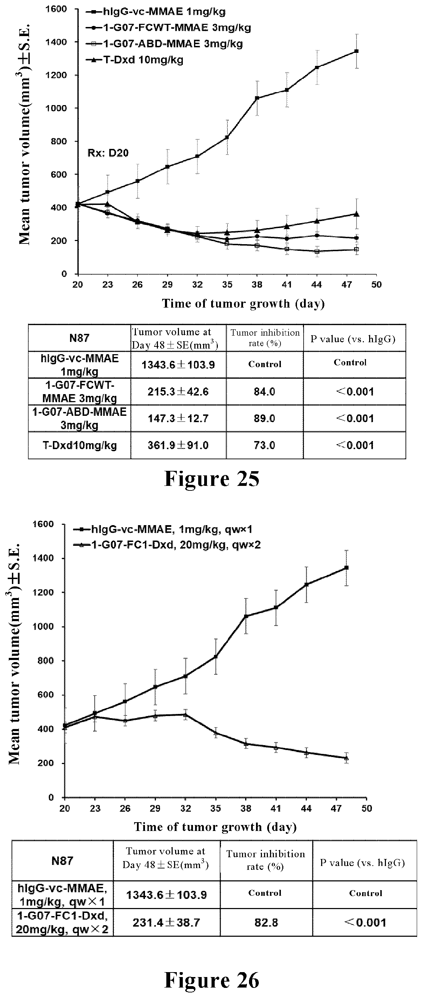

As shown in **Figure 25****,** the HER2 nanobody drug conjugates of the present invention, 1-G07-FC1-MMAE, 1-G07-FCWT-MMAE, and 1-G07-ABD-MMAE that fused with different structural domains, showed significant tumor regression effects on the large volume N87 tumor model at a single dose of 3mg/kg. Among them, 1-G07-ABD-MMAE had the most significant tumor regression effect and was superior to 10mg/kg T-Dxd.

As shown in **Figure 26****,** the HER2 nanobody drug conjugate 1-G07-FC1-Dxd of the present invention, administered intravenously at a dose of 20mg/kg once a week for a total of 2 weeks, exhibited significant tumor regression effects on the large volume N87 tumor model.

In summary, the molecular structure formula and coupling reaction formula of the nanobody conjugate designed by the present invention are shown in **Figures 27** **and** **28****,** and the results of the examples demonstrated excellent anti-tumor effects.

### Amino acid sequence of HER2 nanbody of the present invention

SEQ ID NO.1: 1-C10 CDR1 GLTFRRYE
SEQ ID NO.2: 1-C10 CDR2 ITPASGSR
SEQ ID NO.3: 1-C10 CDR3 NAYWMIPQHIENNY
SEQ ID NO.4: 1-C10 VH 
QVQLVESGGDLVQPGGSLRLSCATSGLTFRRYELKWYRQAPGKERELVAGITPAS
GSRNYADSVKGRFTISRDDAKMTVYLQMNSLEPEDTAVYYCNAYWMIPQHIENNYW
GKGTLVTVSS
SEQ ID NO.5: 2-C07 CDR1 GLTFMRYD
SEQ ID NO.6: 2-C07 CDR2 LDSSSGRT
SEQ ID NO.7: 2-C07 CDR3 NAQWKRIGADY
SEQ ID NO.8: 2-C07 VH 
AVQLVESGGGLVQPGGSLRLSCAASGLTFMRYDINWYRQAPGKERELVAALDSS
SGRTNYADFTEGRFTISRNNAKNSAYLQMNNLKPEDTASYYCNAQWKRIGADYWGQ
GTQVTVFS
SEQ ID NO.9: 1-B05 CDR1 GITFFRHT
SEQ ID NO.10: 1-B05 CDR2 ITGGESTN
SEQ ID NO.11: 1-B05 CDR3 NSKWDAAGVEF
SEQ ID NO.12: 1-B05 VH 
AVQLVESGGGPGGSLRLSCAASGITFFRHTLGWYRQAPGKQRELVASITGGESTN
YADSVKSRFTISRDNAKNMVFLQMNSLKPEDTALYYCNSKWDAAGVEFWGKGTLVT
VSS
SEQ ID NO.13: 1-C09 CDR1 GLTFRRYH
SEQ ID NO.14: 1-C09 CDR2 ISDTGSTT
SEQ ID NO.15: 1-C09 CDR3 NAKWPIIAADW
SEQ ID NO.16: 1-C09 VH 
DVQLVESGGGLVQPGGSLRLSCEASGLTFRRYHFSWYRQPPGKERELVAVISDTG
STTNYTDSVKGRFTISRDLARNTVFLQLNSLKPDDTAVYYCNAKWPIIAADWWGQGT
QVTVSS
SEQ ID NO.17: 2-F03 CDR1 GLTFRRYH
SEQ ID NO.18: 2-F03 CDR2 ISDTGTTN
SEQ ID NO.19: 2-F03 CDR3 NGRWPAVAADW
SEQ ID NO.20: 2-F03 VH 
EVQLVESGGGLVQPGGSLRLSCAASGLTFRRYHMSWYRQAPGKERELVAVISDT
GTTNYAESVKGRFTISRDNSRSTVFLQLNSLKPDDTAVYYCNGRWPAVAADWWGQG
TQVTVSS
SEQ ID NO.21: 1-G07 CDR1 RITFRHYD
SEQ ID NO.22: 1-G07 CDR2 VTNGGVIT
SEQ ID NO.23: 1-G07 CDR3 NAVWVYLQRLTQNKKENDY
SEQ ID NO.24: 1-G07 VH 
QVQLVESGGGKAQPGGSLRLSCVASRITFRHYDLSWYRQAPGQERELVATVTNG
GVITYADSVKGRFTISRDNAKNTVQLQMNNLKPEDTAVYYCNAVWVYLQRLTQNKK
ENDYWGPGTQVTVSS
SEQ ID NO.25: 1-G07-FC1 
QVQLVESGGGKAQPGGSLRLSCVASRITFRHYDLSWYRQAPGQERELVATVTNG
GVITYADSVKGRFTISRDNAKNTVQLQMNNLKPEDTAVYYCNAVWVYLQRLTQNKK
ENDYWGPGTQVTVSSEPKSSDKTHTCPPCPAPEFEGGPSVFLFPPKPKDTLMISRTPEVT
CVVVDVSHEDPEVKFNWYVDGVEVHNAKTKPREEQYNSTYRVVSVLTVLHQDWLNG
KEYKCKVSNKALPASIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEAL
HNHYTQKSLSLSPGK
SEQ ID NO.26: 1-G07-FCWT 
QVQLVESGGGKAQPGGSLRLSCVASRITFRHYDLSWYRQAPGQERELVATVTNG
GVITYADSVKGRFTISRDNAKNTVQLQMNNLKPEDTAVYYCNAVWVYLQRLTQNKK
ENDYWGPGTQVTVSSEPKSSDKTHTCPPCPAPELLGGPSVFLFPPKPKDTLMISRTPEVT
CVVVDVSHEDPEVKFNWYVDGVEVHNAKTKPREEQYNSTYRVVSVLTVLHQDWLNG
KEYKCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEAL
HNHYTQKSLSLSPGK
SEQ ID NO.27: 1-G07-ABD

QVQLVESGGGKAQPGGSLRLSCVASRITFRHYDLSWYRQAPGQERELVATVTNG
GVITYADSVKGRFTISRDNAKNTVQLQMNNLKPEDTAVYYCNAVWVYLQRLTQNKK
ENDYWGPGTQVTVSSGGGGSGGGGSGGGGSQHDEAVDANSLAEAKVLANRELDKYG
VSDYYKNLINNAKTVEGVKALIDEILAALPAAACAAAHHHHHH

All literatures mentioned in the present application are incorporated herein by reference, as though each one is individually incorporated by reference. In addition, it should also be understood that, after reading the above teachings of the present invention, those skilled in the art can make various changes or modifications, and these equivalents also fall in the scope of claims as defined in the appended claims.

## Claims

1. A nanobody targeting HER2, wherein the complementary determining region CDR of the VHH chain of the nanobody is selected from one or more of the following groups:
(1) CDR1 as shown in SEQ ID NO. 1,
CDR2 as shown in SEQ ID NO. 2, and
CDR3 as shown in SEQ ID NO. 3;
or,
(2) CDR1 as shown in SEQ ID NO. 5,
CDR2 as shown in SEQ ID NO. 6, and
CDR3 as shown in SEQ ID NO. 7;
or,
(3) CDR1 as shown in SEQ ID NO. 9,
CDR2 as shown in SEQ ID NO. 10, and
CDR3 as shown in SEQ ID NO. 11;
or,
(4) CDR1 as shown in SEQ ID NO. 13,
CDR2 as shown in SEQ ID NO. 14, and
CDR3 as shown in SEQ ID NO. 15;
or,
(5) CDR1 as shown in SEQ ID NO. 17,
CDR2 as shown in SEQ ID NO. 18, and
CDR3 as shown in SEQ ID NO. 19;
or,
(6) CDR1 as shown in SEQ ID NO. 21,
CDR2 as shown in SEQ ID NO. 22, and
CDR3 as shown in SEQ ID NO. 23.

2. The nanobody according to claim 1, wherein the VHH chain of the nanobody further comprises a framework region (FR).

3. An antibody targeting HER2, wherein the antibody comprises one or more VHH chains of the nanobody targeting HER2 according to claim 2.

4. A multispecific antibody, wherein the multispecific antibody comprises: the nanobody targeting HER2 according to claim 1, or the antibody targeting HER2 according to claim 3.

5. A recombinant protein, wherein the recombinant protein comprises:
(i) the nanobody targeting HER2 according to claim 1, or the antibody targeting HER2 according to claim 2; and
(ii) an optional polypeptide molecule or fragment with therapeutic function;
(iii) an optional functional domain that enhances protein physicochemical properties or drug properties.

6. The recombinant protein according to claim 5, wherein the recombinant protein has the following elements from the N-C end:
A-B or A-Linker-B;
wherein, the element A is the nanobody targeting HER2; the element B is Fc segment, albumin binding domain (ABD), or anti albumin nanobody (HLE);
"-" represents a peptide bond, and Linker represents a linker.

7. The recombinant protein according to claim 5, wherein the VHH chain of the nanobody targeting HER2 is selected from the following group: the amino acid sequence shown in SEQ ID NO.4, SEQ ID NO.8, SEQ ID NO.12, SEQ ID NO.16, SEQ ID NO.20, or SEQ ID NO.24.

8. A CAR construct, wherein the antigen binding region of the CAR construct is the VHH chain of the nanobody according to claim 1.

9. A recombinant immune cell expressing exogenous CAR construct according to claim 6.

10. An immunoconjugate, wherein the immunoconjugate comprising:
(a) an antibody moiety, wherein the antibody moiety is the nanobody targeting HER2 according to claim 1, or the antibody targeting HER2 according to claim 3, or the recombinant protein according to any one of claims 5 to 7; and
(b) a coupling moiety coupled to the nanobody moiety, which is selected from the group consisting of: a detectable label, a drug, a toxin, a cytokine, an enzyme, a protein degrading agent, an oligonucleoxylic acid, and a combination thereof.

11. The immunoconjugate according to claim 10, wherein the protein degrading agent is a degrading agent for tumor associated proteins selected from the group consisting of: EGFR, NF-κB, RIPK2, BCR-ABL, HER2, c-Met, TBK1, CDK, ALK, Akt, CK2, ERK 1/2, FLT3, PI3K, BTK, TRK, Fak, BRD, AR, ER, MetAp-2, BCL-XL, Sirt2, HDAC6, Pirin, SMAD3, ARNT, PCAF/GCN5, Tau, EZH2, IRAK4, STAT3 FRS2, RAS (such as KRAS, HRAS, and NRAS).

12. The immunoconjugate according to claim 10, wherein the immunoconjugate is an antibody drug conjugate ADC with the following molecular formula: 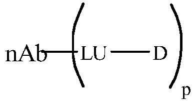 wherein:
nAb is the nanobody targeting HER2,
LU is a linker (also called a connector);
D is a drug;
and the subscript p is a value selected from 1 to 8.

13. The immunoconjugate according to claim 10, wherein the LU is selected from the group consisting of: maleimidocaproyl (MC), maleimide (MAL), succinimidyl 4-(n-maleimidyl methyl) cyclohexane-1-formate) (SMCC) linker to partially connect with the antibody, and comprises one or more of the linkers of valine-citrulline (VC), valine-alanine (VA), glycine-glycine-phenylalanine-glycine (GGFG), alanine-alanine-alanine (AAA), p-aminobenzyloxycarbonyl (PAB), polyethylene glycol (PEG).

14. The immunoconjugate according to claim 10, wherein the D is a compound with antitumor activity selected from the group consisting of:
(i) a tubulin inhibitor, such as maytanin derivatives (DM1, DM4), monomethyl orrestatin E(MMAE), monomethyl orrestatin F(MMAF);
(ii) a toxin that acts on DNA, such as duocarmycin, pyrrolobenzodiazepine (PBD);
(iii) a topoisomerase inhibitor, camptothecin, SN38, Exitecan, Dxd.

15. A pharmaceutical composition, wherein the pharmaceutical composition comprising:
(i) the nanobody targeting HER2 according to claim 1, or the antibody targeting HER2 according to claim 3, or the multispecific antibody according to claim 4, or the recombinant protein according to any one of claims 5 to 7, or the recombinant immune cell according to claim 7, or the immunoconjugate according to any one of claims 10 to 14;
(ii) a pharmaceutically acceptable carrier.

16. Use of an active ingredient selected from the group consisting of: the nanobody targeting HER2 according to claim 1, or the antibody targeting HER2 according to claim 3, or the multispecific antibody according to claim 4, or the recombinant protein according to any one of claims 5 to 7, or the recombinant immune cell according to claim 9, or the immunoconjugate according to any one of claims 10 to 14, and combinations thereof, wherein the active ingredient is used for (a) preparation of a detection reagent, a detection plate or a kit; and/or (b) preparing a drug for the prevention and/or treatment of a HER2-related disease.

17. A method (including diagnostic or non-diagnostic method) for detecting HER2 in a sample *in vitro,* wherein the method comprising the steps of:
(1) contacting the sample with the nanobody targeting HER2 according to claim 1, or the antibody targeting HER2 according to claim 3, or the immunoconjugate according to any one of claims 10 to 14 *in vitro;*
(2) detecting whether an antigen-antibody complex is formed, wherein the formation of the complex indicates the presence of HER2 in the sample.
